# EUROPEAN PATENT APPLICATION

(11) **EP 4 289 826 A1**
(43) Date of publication of application: **13.12.2023**
(21) Application number: 22749046.3
(22) Date of filing: 27.01.2022
(51) Int. Cl.: C07D 263/57, A61K 31/454, A61P 31/16

(54) **SALT AND CRYSTAL FORM OF HA INHIBITOR COMPOUND**

(30) Priority: 04.02.2021 CN 202110157185
(71) Applicant: Sichuan Haisco Pharmaceutical Co., Ltd., Sichuan 611130 (CN)
(72) Inventor: LI, Yao, CHENGDU, Sichuan 611130 (CN); ZHANG, Guobiao, CHENGDU, Sichuan 611130 (CN); ZHANG, Xiaobo, CHENGDU, Sichuan 611130 (CN); TANG, Pingming, CHENGDU, Sichuan 611130 (CN); ZHANG, Chen, CHENGDU, Sichuan 611130 (CN); YAN, Pangke, CHENGDU, Sichuan 611130 (CN)
(74) Representative: Casalonga
(86) International application number: PCT/CN2022/074368
(87) International publication number: WO 2022/166767

(57) **Abstract**

Disclosed are a pharmaceutically acceptable salt of an HA inhibitor (1*S*,2*S*)-2-fluoro-N-(2-(2-(4-((R)-(5-methyl-2H-tetrazol-2-yl)(phenyl)methyl)piperidine-1-formyl)pyridine-4-yl)benzo[d]oxazol-5-yl)cyclopropyl-1-carboxamide, or a hydrate or solvate of a salt thereof, a preparation method therefor, and a use thereof.

## Description

The present application is based on and claims the right of priority for the application with the application no. being CN 202110157185.7 and the filing date being 4 February 2021, and the disclosure of the present application is incorporated herein by reference in its entirety.

### Technical Field

The present application relates to a pharmaceutically acceptable salt of an HA inhibitor compound (1*S*,2*S*)-2-fluoro-N-(2-(2-(4-((*R*)-(5-methyl-2H-tetrazol-2-yl)(phenyl)methyl)piperidine-1-carbonyl)pyridin-4-yl)benzo[d]oxazol-5-yl)cyclopropane-1-carboxamide, or a hydrate or solvate, a crystal form thereof, a preparation method therefor, a pharmaceutical composition thereof and the use thereof in the preparation of a hemagglutinin (HA) inhibitor.

### Background Art

Influenza (flu for short) is an acute respiratory infection caused by influenza viruses and is also a highly infectious and fast-transmitting disease. At present, anti-influenza virus drugs mainly target the hemagglutinin receptor, neuraminidase and matrix protein of the viral envelope.

According to the surface antigen hemagglutinin (HA) and neuraminidase (NA) protein structures and gene characteristics, influenza A viruses are divided into different subtypes. Hemagglutinins that have been discovered currently comprise 18 subtypes (H1 to H18), and neuraminidases that have been discovered currently comprise 10 subtypes (N1 to N10). The entry of a virus into a host cell is the first important step of the initiation of a viral replication cycle. Influenza virus protein hemagglutinins can recognize the potential binding site of sialic acid (SA) (N-acetylneuraminic acid) on glycoproteins of a host cell. The HAs contained in influenza viruses which infect humans have high specificity to α2-6SA. After HAs bind to receptors, the viruses are endocytosed; and the acidic pH of endosomes causes changes on the conformation of HA proteins, thereby regulating the internal fusion of the viruses and recipient cells and releasing RNPs of the viruses into cytoplasm. Therefore, by using HAs as targets and binding to the HAs, the conformational change of HA2 caused by low pH conditions is inhibited, thereby inhibiting the process of fusion of viral envelopes with host endosomal membranes, which has become a new anti-influenza virus strategy. Currently, there are multiple vaccines for HAs in the clinical stage, such as CR9114 (WO 2013/007770) and CR6261 (WO 2008/028946). Small molecule compounds with different structural features for the treatment of influenza have also been reported in the literature. A series of benzisoxazole compounds for the treatment of influenza are reported in WO 2012/144752.

### Summary of the Invention

The present application provides a salt having the following structure (labelled as compound A), or a hydrate, a solvate or a crystal form of a salt thereof,

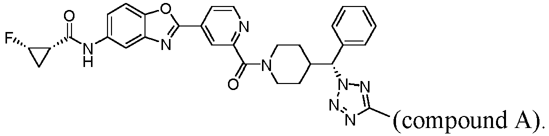

The salt of compound A, and the hydrate, solvate or crystal form of the salt thereof have better solubility and stability than a free base compound, can be very stable in a diluent (solvent) and are capable of resisting high temperature, high humidity and strong light during the preparation of a medicament or a composition thereof (which are suitable for the preparation of pharmaceutical dosage forms), and also have better pharmacokinetics and bioavailability than a free base compound.

In particular, the present application provides a salt of a compound represented by formula (I), or a hydrate or solvate of a salt thereof:

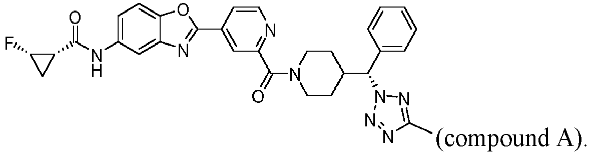

The salt is selected from hydrochloride, hydrobromide, 2-naphthalenesulfonate, benzenesulfonate, methanesulfonate, p-toluenesulfonate, hemi-1,5-naphthalene disulfonate, succinate, citrate or malate.

Further, the salt of compound A is selected from hydrochloride, hydrobromide, 2-naphthalenesulfonate or hemi-1,5-naphthalene disulfonate, preferably the crystal form thereof.

Furthermore, the salt of compound A is the hemi-1,5-naphthalene disulfonate.

The present application provides the salt of compound A or the hydrate or solvate of the salt thereof, wherein the salt has a structure selected from

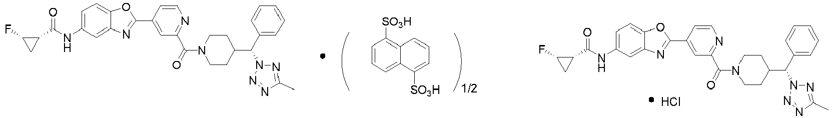

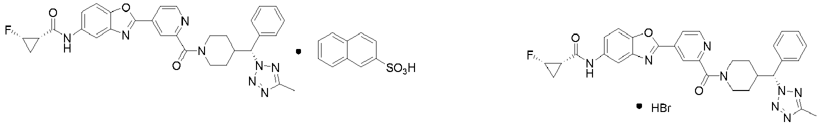

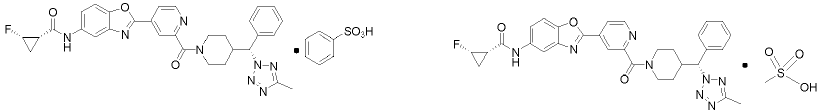

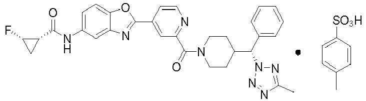

The present application further provides a method for preparing the hemi-1,5-naphthalene disulfonate of compound A, comprising the steps of
(1) dissolving amorphous compound A in solvent 1;
(2) dissolving 1,5-naphthalene disulfonic acid in solvent 2;
(3) adding dropwise a solution obtained in step (2) to a solution obtained in step (1) at room temperature with stirring; and stirring a reaction;
wherein the solvent 1 and solvent 2 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 1 is selected from one of isopropanol, acetone or tetrahydrofuran, and the solvent 2 is a single-solvent system or a double-solvent mixed system, wherein the single-solvent system is selected from one of isopropanol, acetone or tetrahydrofuran, and the double-solvent mixed system is a tetrahydrofuran-water mixed liquid.

The present application further provides a method for preparing the 2-naphthalenesulfonate of compound A, comprising the steps of
(1) dissolving amorphous compound A in solvent 3;
(2) dissolving 2-naphthalenesulfonic acid in solvent 4;
(3) adding dropwise a solution obtained in step (2) to a solution obtained in step (1) at room temperature with stirring; and stirring a reaction;

wherein the solvent 3 and solvent 4 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 3 is selected from one of isopropanol, acetone, tetrahydrofuran, isopropyl acetate, 1,4-dioxane, toluene or dimethyl sulfoxide, and the solvent 4 is a single-solvent system or a double-solvent mixed system, wherein the single-solvent system is selected from one of isopropanol, acetone, tetrahydrofuran, isopropyl acetate, 1,4-dioxane, toluene or dimethyl sulfoxide, and the double-solvent mixed system is a toluene-methanol mixed liquid;
further, the solvent 3 and solvent 4 are identical.

The present application further provides a method for preparing the hydrochloride of compound A, comprising the steps of
(1) dissolving amorphous compound A in solvent 5;
(2) dissolving hydrochloric acid in solvent 6;
(3) adding dropwise a solution obtained in step (2) to a solution obtained in step (1) at room temperature with stirring; and stirring a reaction;
wherein the solvent 5 and solvent 6 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 5 and solvent 6 are each independently selected from one of isopropanol, acetone and tetrahydrofuran, and furthermore, the solvent 5 and solvent 6 are identical and each independently selected from one of isopropanol, acetone and tetrahydrofuran.

The present application further provides a method for preparing the hydrobromide of compound A, comprising the steps of
(1) dissolving amorphous compound A in solvent 7;
(2) dissolving hydrobromic acid in solvent 8;
(3) adding dropwise a solution obtained in step (2) to a solution obtained in step (1) at room temperature with stirring; and stirring a reaction;
wherein the solvent 7 and solvent 8 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 7 and solvent 8 are each independently selected from one of isopropanol, acetone and tetrahydrofuran, and furthermore, the solvent 7 and solvent 8 are identical and each independently selected from one of isopropanol, acetone and tetrahydrofuran.

The present application further provides a method for preparing the benzenesulfonate of compound A, comprising the steps of
(1) dissolving amorphous compound A in solvent 9;
(2) dissolving benzenesulfonic acid in solvent 10;
(3) adding dropwise a solution obtained in step (2) to a solution obtained in step (1) at room temperature with stirring; and stirring a reaction;
wherein the solvent 9 and solvent 10 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 9 is selected from one of isopropanol, acetone, tetrahydrofuran, isopropyl acetate, 1,4-dioxane, toluene or dimethyl sulfoxide, and the solvent 10 is a single-solvent system or a double-solvent mixed system, wherein the single-solvent system is selected from one of isopropanol, acetone, tetrahydrofuran, isopropyl acetate, 1,4-dioxane, toluene or dimethyl sulfoxide, and the double-solvent mixed system is a toluene-methanol mixed liquid; further, the solvent 9 and solvent 10 are identical.

The present application further provides a method for preparing the p-toluenesulfonate of compound A, comprising the steps of
(1) dissolving amorphous compound A in solvent 11;
(2) dissolving p-toluenesulfonic acid in solvent 12;
(3) adding dropwise a solution obtained in step (2) to a solution obtained in step (1) at room temperature with stirring; and carrying out stirring, crystallization, centrifugation and drying;
wherein the solvent 11 and solvent 12 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 11 is selected from one of isopropanol, acetone, tetrahydrofuran, isopropyl acetate, 1,4-dioxane, toluene or dimethyl sulfoxide, and the solvent 12 is a single-solvent system or a double-solvent mixed system, wherein the single-solvent system is selected from one of isopropanol, acetone, tetrahydrofuran, isopropyl acetate, 1,4-dioxane, toluene or dimethyl sulfoxide, and the double-solvent mixed system is a toluene-methanol mixed liquid; further, the solvent 11 and solvent 12 are identical.

The present application further provides a method for preparing the p-methanesulfonate of compound A, comprising the steps of
(1) dissolving amorphous compound A in solvent 13;
(2) dissolving methanesulfonic acid in solvent 14;
(3) adding dropwise a solution obtained in step (2) to a solution obtained in step (1) at room temperature with stirring; and carrying out stirring, crystallization, centrifugation and drying;
wherein the solvent 13 and solvent 14 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 13 is selected from one of isopropanol, acetone, tetrahydrofuran, isopropyl acetate, 1,4-dioxane, toluene or dimethyl sulfoxide, and the solvent 14 is a single-solvent system or a double-solvent mixed system, wherein the single-solvent system is selected from one of isopropanol, acetone, tetrahydrofuran, isopropyl acetate, 1,4-dioxane, toluene or dimethyl sulfoxide, and the double-solvent mixed system is a toluene-methanol mixed liquid; further, the solvent 13 and solvent 14 are identical.

The present application further provides a method for preparing the acetate of compound A, comprising the steps of
(1) dissolving amorphous compound A in solvent 15;
(2) dissolving acetic acid in solvent 16;
(3) adding dropwise a solution obtained in step (2) to a solution obtained in step (1) at room temperature with stirring; and carrying out stirring, crystallization, centrifugation and drying;
wherein the solvent 15 and solvent 16 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 15 is selected from a single-solvent system or a double-solvent mixed system, wherein the single-solvent system is selected from one of isopropanol, methanol, acetic acid, acetone and tetrahydrofuran; and the solvent 16 is a single-solvent system or a double-solvent mixed system, wherein the single-solvent system is selected from one of isopropanol, acetic acid, acetone, tetrahydrofuran and methanol, and the double-solvent mixed system comprises a first solvent that is acetic acid and a second solvent that is selected from one of water, n-heptane and methyl tert-butyl ether.

The present application further provides a method for preparing the fumarate of compound A, comprising the steps of
(1) dissolving amorphous compound A in solvent 17;
(2) dissolving fumaric acid in solvent 18;
(3) adding dropwise a solution obtained in step (2) to a solution obtained in step (1) at room temperature with stirring; and carrying out stirring, crystallization, centrifugation and drying; wherein the solvent 17 and solvent 18 are each independently selected from one of or a mixed solvent of two or more of an aromatic hydrocarbon, an ester solvent, an ether solvent, an aliphatic hydrocarbon solvent, an alicyclic hydrocarbon solvent, an alcohol solvent, a glycol derivative solvent, a halogenated hydrocarbon solvent, a ketone solvent, acetonitrile and water; further, the solvent 17 and solvent 18 are selected from one of isopropanol, acetone, tetrahydrofuran, ethanol, methanol, isopropyl acetate, 1,4-dioxane, toluene or dimethyl sulfoxide; further, the solvent 17 and solvent 18 are identical.

With the same method as the fumarate of compound A, the malonate, succinate, benzoate, citrate, malate and L-tartrate can be prepared by selecting the corresponding acids.

The method for preparing the salt of compound A of the present application may further involve adding an anti-solvent, wherein the anti-solvent can be selected from one of isopropyl ether, diethyl ether, water and n-heptane.

In the method for preparing the salt of compound A of the present application, the molar ratio of compound A to an acid is 1 : 5-1 : 1.1, further 1 : 2-1 : 1.2, and more further 1 : 1.5-1 : 1.2.

The method for preparing the salt of compound A of the present application is performed at normal temperature, further 15°C-30°C.

The present application also relates to a hemi-1,5-naphthalene disulfonate of compound A, which is in the form of a crystal (crystal form I of hemi-1,5-naphthalene disulfonate) and has an X-ray powder diffraction pattern comprising characteristic diffraction peaks at 5.3° ± 0.2°, 13.4° ± 0.2°, 17.4° ± 0.2°, 18.5° ± 0.2°, 20.4° ± 0.2° and 23.6° ± 0.2° 2θ, as determined by using Cu-Kα radiation.

Further, the crystal form I of the hemi-1,5-naphthalene disulfonate of compound A provided by the present application has an X-ray powder diffraction pattern further comprising characteristic diffraction peaks at 7.0° ± 0.2°, 9.8° ± 0.2°, 10.6° ± 0.2°, 12.7° ± 0.2°, 14.8° ± 0.2°, 22.2° ± 0.2° and 23.1° ± 0.2° 2θ.

Furthermore, the crystal form I of the hemi-1,5-naphthalene disulfonate of compound A provided by the present application has an X-ray powder diffraction pattern further comprising characteristic diffraction peaks at 14.1° ± 0.2°, 16.0° ± 0.2° and 21.5° ± 0.2° 2θ.

Furthermore, the crystal form I of the hemi-1,5-naphthalene disulfonate of compound A provided by the present application has an X-ray powder diffraction pattern further comprising characteristic diffraction peaks at 9.8° ± 0.2°, 11.6° ± 0.2°, 16.6° ± 0.2°, 17.9° ± 0.2°, 19.2° ± 0.2° and 27.6° ± 0.2° 2θ.

Furthermore, the crystal form I of the hemi-1,5-naphthalene disulfonate of compound A provided by the present application has an X-ray powder diffraction pattern further comprising characteristic diffraction peaks at 18.9° ± 0.2°, 21.8° ± 9.2°, 24.9° ± 0.2°, 27.3° ± 0.2°, 27.9° ± 0.2°, 28.33° ± 0.2°, 29.0° ± 0.2° and 33.4° ± 0.2° 2θ.

Table 1 shows 2θ values and corresponding intensities in the X-ray powder diffraction pattern of the crystal form I of the hemi-1,5-naphthalene disulfonate of compound A provided by the present application, wherein the error range of 2θ is ± 0.2°.

**Table 1. 2θ values and corresponding intensities in XRPD pattern of crystal form I of hemi-1,5-naphthalene disulfonate of compound A**

| **2-Theta** | **d** | **Height** | **1%** | **Area** | **1%** |
|---|---|---|---|---|---|
| 5.305 | 16.6455 | 2212 | 100 | 18185 | 74.1 |
| 7.038 | 12.5499 | 696 | 31.5 | 4868 | 19.8 |
| 8.787 | 10.0552 | 87 | 3.9 | 468 | 1.9 |
| 9.823 | 8.9969 | 484 | 21.9 | 3360 | 13.7 |
| 10.624 | 8.3203 | 551 | 24.9 | 5101 | 20.8 |
| 11.57 | 7.6418 | 107 | 4.8 | 725 | 3 |
| 12.661 | 6.9859 | 835 | 37.7 | 6116 | 24.9 |
| 13.362 | 6.621 | 1424 | 64.4 | 16895 | 68.9 |
| 14.061 | 6.2931 | 548 | 24.8 | 4059 | 16.5 |
| 14.424 | 6.1358 | 145 | 6.6 | 1642 | 6.7 |
| 14.805 | 5.9786 | 889 | 40.2 | 8420 | 34.3 |
| 15.364 | 5.7623 | 185 | 8.4 | 1199 | 4.9 |
| 15.985 | 5.5399 | 554 | 25 | 7337 | 29.9 |
| 16.565 | 5.3471 | 377 | 17 | 3210 | 13.1 |
| 17.428 | 5.0843 | 1560 | 70.5 | 13366 | 54.5 |
| 17.925 | 4.9444 | 468 | 21.2 | 5129 | 20.9 |
| 18.506 | 4.7904 | 2139 | 96.7 | 21406 | 87.3 |
| 18.943 | 4.681 | 336 | 15.2 | 7868 | 32.1 |
| 19.206 | 4.6175 | 536 | 24.2 | 7413 | 30.2 |
| 20.427 | 4.3442 | 1917 | 86.7 | 24529 | 100 |
| 21.487 | 4.1321 | 574 | 25.9 | 14636 | 59.7 |
| 21.788 | 4.0757 | 214 | 9.7 | 2341 | 9.5 |
| 22.247 | 3.9927 | 800 | 36.2 | 7620 | 31.1 |
| 23.109 | 3.8457 | 965 | 43.6 | 19204 | 78.3 |
| 23.647 | 3.7593 | 1254 | 56.7 | 22666 | 92.4 |
| 24.948 | 3.5662 | 251 | 11.3 | 5183 | 21.1 |
| 25.415 | 3.5018 | 96 | 4.3 | 454 | 1.9 |
| 26.57 | 3.352 | 137 | 6.2 | 1305 | 5.3 |
| 27.311 | 3.2627 | 232 | 10.5 | 8627 | 35.2 |
| 27.571 | 3.2326 | 434 | 19.6 | 15320 | 62.5 |
| 27.871 | 3.1985 | 369 | 16.7 | 18547 | 75.6 |
| 28.337 | 3.1469 | 145 | 6.6 | 746 | 3 |
| 29.033 | 3.073 | 153 | 6.9 | 3527 | 14.4 |
| 29.594 | 3.016 | 61 | 2.8 | 966 | 3.9 |
| 30.694 | 2.9104 | 57 | 2.6 | 818 | 3.3 |
| 32.41 | 2.7601 | 71 | 3.2 | 2277 | 9.3 |
| 33.429 | 2.6783 | 124 | 5.6 | 2700 | 11 |
| 34.108 | 2.6265 | 67 | 3 | 928 | 3.8 |
| 34.347 | 2.6087 | 54 | 2.4 | 924 | 3.8 |

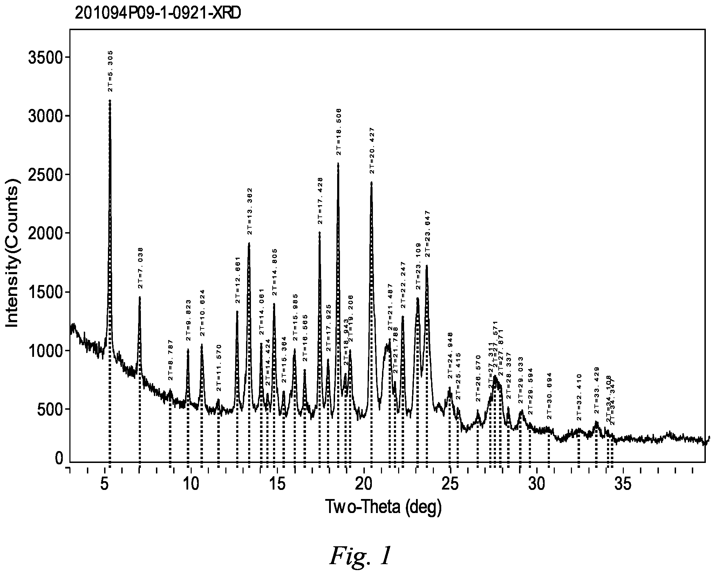

Further, the crystal form I of the hemi-1,5-naphthalene disulfonate of compound A provided by the present application has an X-ray powder diffraction pattern substantially as shown in Figure 1.

The crystal form I of the hemi-1,5-naphthalene disulfonate of compound A provided by the present application has a differential scanning calorimetry (DSC) pattern showing an endothermic curve, wherein Tₛₜₐᵣₜ = 188.78°C, Tₚₑₐₖ = 198.44°C and △H = 46.09 J/g.

The crystal form I of the hemi-1,5-naphthalene disulfonate of compound A provided by the present application has a differential scanning calorimetry (DSC) pattern showing a melting point of 188.78°C.

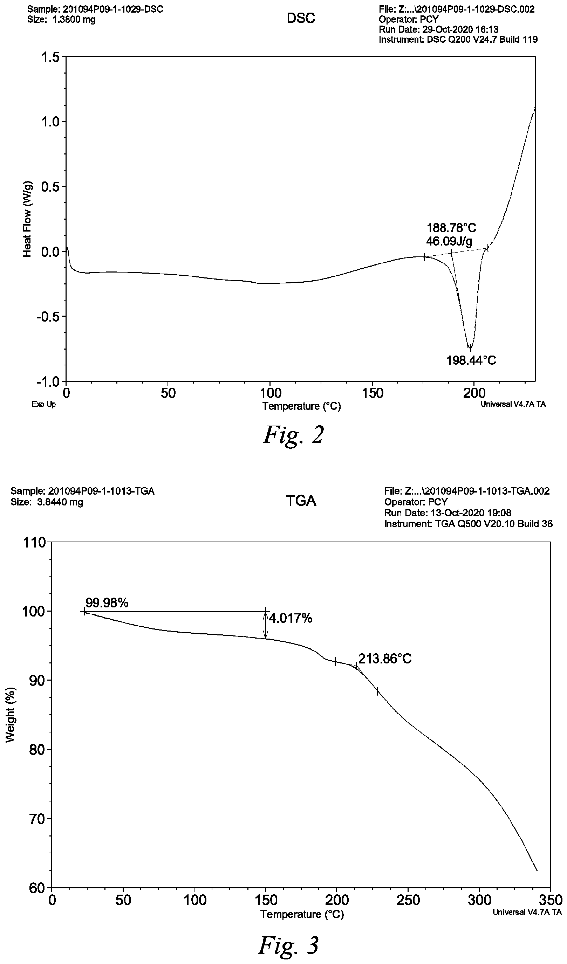

The crystal form I of the hemi-1,5-naphthalene disulfonate of compound A provided by the present application has a differential scanning calorimetry (DSC) pattern substantially as shown in Figure 2.

The crystal form I of the hemi-1,5-naphthalene disulfonate of compound A provided by the present application has a thermogravimetric analysis (TGA) curve showing a weight loss of 4.017% below 150°C and showing a decomposition temperature of 213.86°C.

The crystal form I of the hemi-1,5-naphthalene disulfonate of compound A provided by the present application has a thermogravimetric analysis (TGA) curve substantially as shown in Figure 3.

The crystal form I of the hemi-1,5-naphthalene disulfonate of compound A provided by the present application is an anhydride.

The present application also provides a hydrochloride of compound A, which is in the form of a crystal (crystal form I of hydrochloride) and has an X-ray powder diffraction pattern comprising characteristic diffraction peaks at 5.9° ± 0.2°, 11.2° ± 0.2°, 11.7° ± 0.2°, 17.6° ± 0.2°, 18.2° ± 0.2°, 21.9° ± 0.2° and 26.8° ± 0.2° 2θ, as determined by using Cu-Kα radiation.

Further, the crystal form I of the hydrochloride of compound A provided by the present application has an X-ray powder diffraction pattern further comprising characteristic diffraction peaks at 7.1° ± 0.2°, 16.3° ± 0.2°, 18.6° ± 0.2°, 22.3° ± 0.2° and 23.8° ± 0.2° 2θ.

Furthermore, the crystal form I of the hydrochloride of compound A provided by the present application has an X-ray powder diffraction pattern further comprising characteristic diffraction peaks at 13.3° ± 0.2°, 14.2° ± 0.2°, 15.7° ± 0.2°, 20.3° ± 0.2°, 21.3° ± 0.2°, 24.8° ± 0.2°, 25.4° ± 0.2°, 27.2° ± 0.2° and 27.7° ± 0.2° 2θ.

Furthermore, the crystal form I of the hydrochloride of compound A provided by the present application has an X-ray powder diffraction pattern further comprising characteristic diffraction peaks at 6.6 ± 0.2°, 8.1 ± 0.2°, 10.3 ± 0.2°, 12.9 ± 0.2°, 15.9 ± 0.2°, 21.3 ± 0.2°, 23.0 ± 0.2° and 23.6 ± 0.2° 2θ.

Furthermore, the crystal form I of the hydrochloride of compound A provided by the present application has an X-ray powder diffraction pattern further comprising characteristic diffraction peaks at 21.0 ± 0.2°, 24.3 ± 0.2°, 24.8 ± 0.2°, 26.4 ± 0.2°, 26.1 ± 0.2°, 27.2 ± 0.2°, 28.8 ± 0.2° and 29.8 ± 0.2° 2θ.

Table 2 shows 2θ values and corresponding intensities in the X-ray powder diffraction pattern of the crystal form I of the hydrochloride of compound A provided by the present application, wherein the error range of 2θ is ± 0.2°.

**Table 2. 2θ values and corresponding intensities in XRPD pattern of crystal form I of hydrochloride of compound A**

| **2-Theta** | **d** | **Height** | **I%** | **Area** | **I%** |
|---|---|---|---|---|---|
| 5.897 | 14.9741 | 5151 | 100 | 40269 | 100 |
| 6.648 | 13.2845 | 254 | 4.9 | 1730 | 4.3 |
| 7.122 | 12.4014 | 1436 | 27.9 | 9631 | 23.9 |
| 8.126 | 10.871 | 88 | 1.7 | 642 | 1.6 |
| 10.308 | 8.5748 | 214 | 4.2 | 1181 | 2.9 |
| 11.224 | 7.8767 | 1700 | 33 | 13898 | 34.5 |
| 11.743 | 7.5296 | 2289 | 44.4 | 19229 | 47.8 |
| 12.443 | 7.1075 | 96 | 1.9 | 571 | 1.4 |
| 12.861 | 6.8774 | 247 | 4.8 | 1126 | 2.8 |
| 13.263 | 6.6702 | 581 | 11.3 | 3851 | 9.6 |
| 13.802 | 6.4108 | 70 | 1.4 | 710 | 1.8 |
| 14.224 | 6.2215 | 433 | 8.4 | 2314 | 5.7 |
| 15.305 | 5.7846 | 194 | 3.8 | 3432 | 8.5 |
| 15.661 | 5.6538 | 807 | 15.7 | 11606 | 28.8 |
| 15.946 | 5.5532 | 654 | 12.7 | 6413 | 15.9 |
| 16.346 | 5.4185 | 1035 | 20.1 | 12922 | 32.1 |
| 17.628 | 5.027 | 1656 | 32.1 | 16862 | 41.9 |
| 18.203 | 4.8694 | 1448 | 28.1 | 12001 | 29.8 |
| 18.567 | 4.7749 | 717 | 13.9 | 5345 | 13.3 |
| 19.402 | 4.5712 | 80 | 1.6 | 210 | 0.5 |
| 19.934 | 4.4504 | 92 | 1.8 | 336 | 0.8 |
| 20.328 | 4.3651 | 638 | 12.4 | 4269 | 10.6 |
| 20.636 | 4.3007 | 150 | 2.9 | 2138 | 5.3 |
| 20.951 | 4.2366 | 305 | 5.9 | 4050 | 10.1 |
| 21.267 | 4.1744 | 589 | 11.4 | 6203 | 15.4 |
| 21.867 | 4.0611 | 1221 | 23.7 | 13710 | 34 |
| 22.269 | 3.9888 | 905 | 17.6 | 17475 | 43.4 |
| 23.026 | 3.8593 | 222 | 4.3 | 2134 | 5.3 |
| 23.588 | 3.7686 | 584 | 11.3 | 18729 | 46.5 |
| 23.789 | 3.7373 | 1081 | 21 | 16746 | 41.6 |
| 24.25 | 3.6672 | 323 | 6.3 | 5624 | 14 |
| 24.83 | 3.5828 | 576 | 11.2 | 11094 | 27.5 |
| 25.408 | 3.5026 | 537 | 10.4 | 7688 | 19.1 |
| 25.668 | 3.4677 | 117 | 2.3 | 895 | 2.2 |
| 26.13 | 3.4075 | 356 | 6.9 | 5506 | 13.7 |
| 26.37 | 3.377 | 326 | 6.3 | 6092 | 15.1 |
| 26.827 | 3.3205 | 1286 | 25 | 15713 | 39 |
| 27.171 | 3.2793 | 774 | 15 | 12062 | 30 |
| 27.713 | 3.2163 | 777 | 15.1 | 8536 | 21.2 |
| 28.828 | 3.0944 | 204 | 4 | 2139 | 5.3 |
| 29.256 | 3.0501 | 144 | 2.8 | 4790 | 11.9 |
| 29.832 | 2.9925 | 448 | 8.7 | 6568 | 16.3 |
| 30.534 | 2.9253 | 73 | 1.4 | 259 | 0.6 |
| 30.873 | 2.894 | 92 | 1.8 | 3898 | 9.7 |
| 31.33 | 2.8527 | 157 | 3 | 6508 | 16.2 |
| 32.63 | 2.742 | 94 | 1.8 | 1731 | 4.3 |
| 33.309 | 2.6876 | 65 | 1.3 | 2025 | 5 |
| 35.654 | 2.516 | 125 | 2.4 | 1568 | 3.9 |
| 36.213 | 2.4785 | 252 | 4.9 | 5547 | 13.8 |
| 37.342 | 2.4061 | 55 | 1.1 | 1346 | 3.3 |
| 39.394 | 2.2854 | 67 | 1.3 | 1151 | 2.9 |

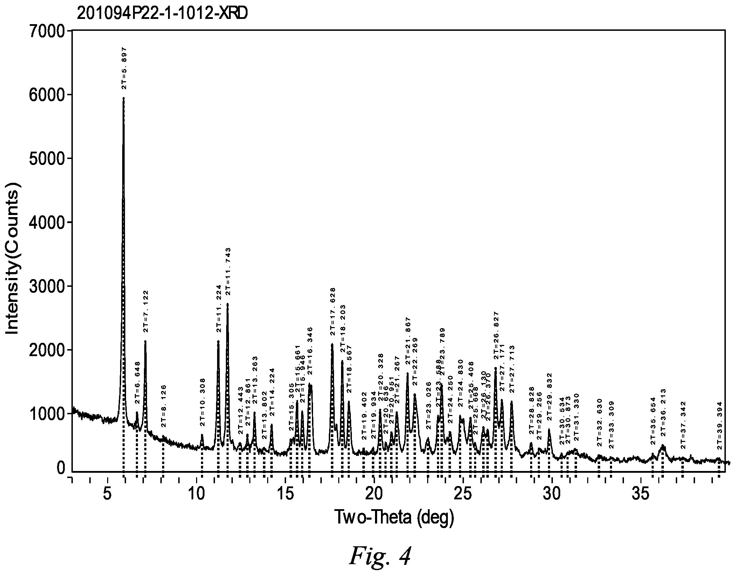

Further, the crystal form I of the hydrochloride of compound A provided by the present application has an X-ray powder diffraction pattern substantially as shown in Figure 4.

The crystal form I of the hydrochloride of compound A provided by the present application has a differential scanning calorimetry (DSC) pattern showing an endothermic curve, wherein Tₛₜₐᵣₜ = 118.97°C, Tₚₑₐₖ = 126.16°C and △H = 23.18 J/g.

Further, the crystal form I of the hydrochloride of compound A provided by the present application has a differential scanning calorimetry (DSC) pattern showing a melting point of 118.97°C.

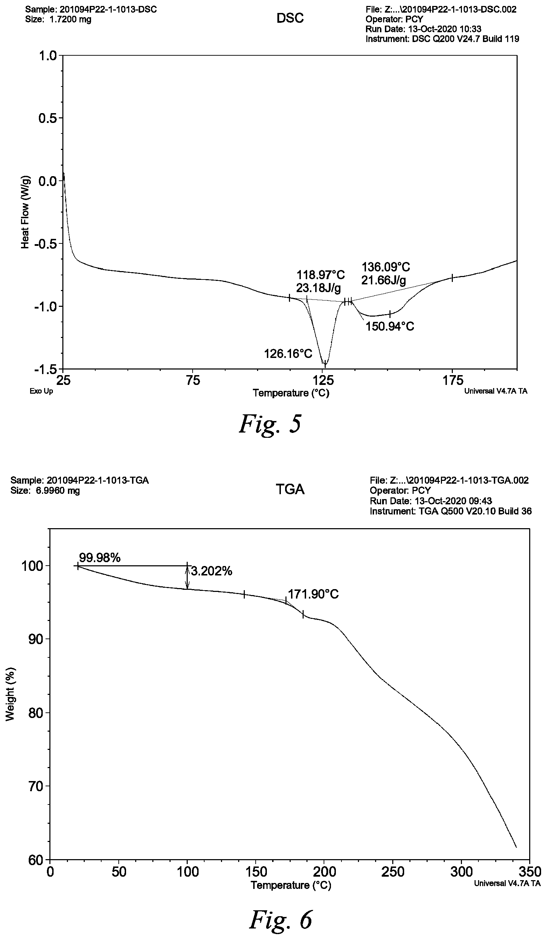

Further, the crystal form I of the hydrochloride of compound A provided by the present application has a differential scanning calorimetry pattern substantially as shown in Figure 5.

The crystal form I of the hydrochloride of compound A provided by the present application has a thermogravimetric analysis (TGA) curve showing a weight loss of 3.202% below 150°C and showing a decomposition temperature of 171.90°C.

Further, the crystal form I of the hydrochloride of compound A provided by the present application has a thermogravimetric analysis (TGA) curve substantially as shown in Figure 6.

The crystal form of the hydrochloride of compound A provided by the present application may exist in the form of a solvate.

The present application also provides a hydrobromide of compound A, which is in the form of a crystal (crystal form I of hydrobromide) and has an X-ray powder diffraction pattern comprising characteristic diffraction peaks at 6.0° ± 0.2°, 7.2° ± 0.2°, 9.0° ± 0.2°, 12.0° ± 0.2°, 14.8° ± 0.2° and 17.6° ± 0.2° 2θ, as determined by using Cu-Kα radiation.

Further, the crystal form I of the hydrobromide of compound A in the present application has an X-ray powder diffraction pattern further comprising characteristic diffraction peaks at 17.3° ± 0.2°, 18.0° ± 0.2°, 21.2° ± 0.2°, 21.5° ± 0.2°, 24.2° ± 0.2° and 26.5° ± 0.2° 2θ.

Further, the crystal form I of the hydrobromide of compound A in the present application has an X-ray powder diffraction pattern further comprising characteristic diffraction peaks at 16.9° ± 0.2°, 18.6° ± 0.2°, 19.0° ± 0.2°, 20.2° ± 0.2° and 28.0° ± 0.2° 2θ.

Further, the crystal form I of the hydrobromide of compound A in the present application has an X-ray powder diffraction pattern further comprising characteristic diffraction peaks at 14.4° ± 0.2°, 20.4° ± 0.2°, 22.1° ± 0.2°, 22.5° ± 0.2°, 23.7° ± 0.2°, 24.8° ± 0.2°, 27.1° ± 0.2° and 28.4° ± 0.2° 2θ.

Further, the crystal form I of the hydrobromide of compound A in the present application has an X-ray powder diffraction pattern further comprising characteristic diffraction peaks at 26.1° ± 0.2° and 27.1° ± 0.2° 2θ.

Table 3 shows 2θ values and corresponding intensities in the X-ray powder diffraction pattern of the crystal form I of the hydrobromide of compound A provided by the present application, wherein the error range of 2θ is ± 0.2°.

**Table 3. 2θ values and corresponding intensities in XRPD pattern of crystal form I of hydrobromide of compound A**

| **2-Theta** | **d** | **Height** | **I%** | **Area** | **I%** |
|---|---|---|---|---|---|
| 6.022 | 14.6654 | 7284 | 100 | 80860 | 100 |
| 7.241 | 12.1978 | 753 | 10.3 | 6205 | 7.7 |
| 8.985 | 9.8343 | 649 | 8.9 | 6034 | 7.5 |
| 11.983 | 7.3796 | 3128 | 42.9 | 41296 | 51.1 |
| 14.445 | 6.1269 | 168 | 2.3 | 2006 | 2.5 |
| 14.804 | 5.9791 | 837 | 11.5 | 10793 | 13.3 |
| 16.871 | 5.2508 | 332 | 4.6 | 3177 | 3.9 |
| 17.345 | 5.1083 | 517 | 7.1 | 16231 | 20.1 |
| 17.604 | 5.0337 | 883 | 12.1 | 18607 | 23 |
| 17.965 | 4.9335 | 355 | 4.9 | 3587 | 4.4 |
| 18.649 | 4.7542 | 327 | 4.5 | 2833 | 3.5 |
| 19.025 | 4.661 | 251 | 3.4 | 4060 | 5 |
| 20.248 | 4.382 | 351 | 4.8 | 5991 | 7.4 |
| 21.245 | 4.1786 | 596 | 8.2 | 10662 | 13.2 |
| 21.549 | 4.1203 | 414 | 5.7 | 5856 | 7.2 |
| 22.143 | 4.0112 | 226 | 3.1 | 3559 | 4.4 |
| 22.528 | 3.9435 | 264 | 3.6 | 4068 | 5 |
| 23.686 | 3.7533 | 287 | 3.9 | 3871 | 4.8 |
| 24.192 | 3.6759 | 490 | 6.7 | 18141 | 22.4 |
| 24.807 | 3.5862 | 320 | 4.4 | 7878 | 9.7 |
| 26.068 | 3.4155 | 98 | 1.3 | 1171 | 1.4 |
| 26.531 | 3.3569 | 480 | 6.6 | 17226 | 21.3 |
| 27.069 | 3.2913 | 309 | 4.2 | 6350 | 7.9 |
| 28.032 | 3.1805 | 358 | 4.9 | 6097 | 7.5 |
| 28.408 | 3.1392 | 150 | 2.1 | 4097 | 5.1 |
| 29.024 | 3.074 | 78 | 1.1 | 903 | 1.1 |
| 29.534 | 3.022 | 105 | 1.4 | 1041 | 1.3 |
| 29.876 | 2.9883 | 89 | 1.2 | 719 | 0.9 |
| 30.572 | 2.9217 | 115 | 1.6 | 1393 | 1.7 |
| 31.331 | 2.8526 | 80 | 1.1 | 785 | 1 |
| 32.093 | 2.7866 | 144 | 2 | 2973 | 3.7 |
| 33.735 | 2.6547 | 66 | 0.9 | 1843 | 2.3 |
| 35.412 | 2.5327 | 65 | 0.9 | 1256 | 1.6 |
| 37.652 | 2.387 | 108 | 1.5 | 3020 | 3.7 |

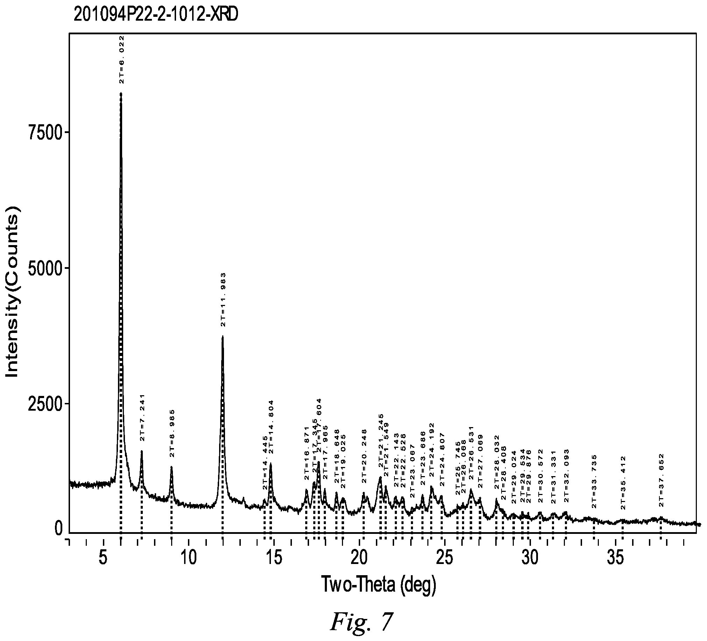

Further, the crystal form I of the hydrobromide of compound A provided by the present application has an X-ray powder diffraction pattern substantially as shown in Figure 7.

The crystal form I of the hydrobromide of compound A provided by the present application has a differential scanning calorimetry (DSC) pattern showing an endothermic curve, wherein Tₛₜₐᵣₜ = 179.68°C, Tₚₑₐₖ = 187.40°C and △H = 8.189 J/g.

Further, the crystal form I of the hydrobromide of compound A provided by the present application has a differential scanning calorimetry (DSC) pattern showing a melting point of 179.68°C.

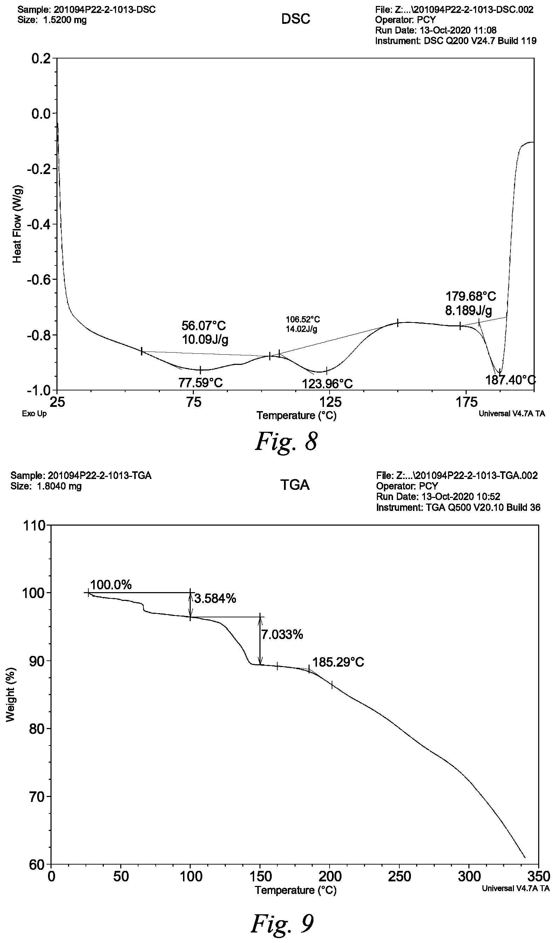

Further, the crystal form I of the hydrobromide of compound A provided by the present application has a differential scanning calorimetry (DSC) pattern substantially as shown in Figure 8.

The crystal form I of the hydrobromide of compound A provided by the present application has a thermogravimetric analysis (TGA) curve showing a weight loss of 3.584% below 100°C and a weight loss of 7.033% between 100°C-150°C and showing a decomposition temperature of 185.29°C.

Further, the crystal form I of the hydrobromide of compound A provided by the present application has a thermogravimetric analysis (TGA) curve substantially as shown in Figure 9.

The present application also relates to a hydrobromide of compound A, which is in the form of a crystal (crystal form II of hydrobromide) and has an X-ray powder diffraction pattern comprising characteristic diffraction peaks at 6.5° ± 0.2°, 7.3° ± 0.2°, 12.2° ± 0.2°, 12.9° ± 0.2° and 16.0° ± 0.2° 2θ, as determined by using Cu-Kα radiation.

Further, the crystal form II of the hydrobromide of compound A in the present application has an X-ray powder diffraction pattern further comprising characteristic diffraction peaks at 6.1° ± 0.2°, 17.4° ± 0.2°, 18.3° ± 0.2°, 20.4° ± 0.2°, 22.4° ± 0.2°, 24.8° ± 0.2° and 28.2° ± 0.2° 2θ.

Further, the crystal form II of the hydrobromide of compound A in the present application has an X-ray powder diffraction pattern further comprising characteristic diffraction peaks at 13.8° ± 0.2°, 14.5° ± 0.2°, 15.2° ± 0.2°, 19.1° ± 0.2°, 19.9° ± 0.2°, 21.4° ± 0.2°, 21.8° ± 0.2°, 23.1° ± 0.2°, 23.6° ± 0.2°, 25.5° ± 0.2°, 26.0° ± 0.2° and 26.5° ± 0.2° 2θ.

Table 4 shows 2θ values and corresponding intensities in the X-ray powder diffraction pattern of the crystal form II of the hydrobromide of compound A provided by the present application, wherein the error range of 2θ is ± 0.2°.

**Table 4. 2θ values and corresponding intensities in XRPD pattern of crystal form II of hydrobromide of compound A**

| **2-Theta** | **d** | **Height** | **I%** | **Area** | **I%** |
|---|---|---|---|---|---|
| 2-Theta | d | Height | I% | Area | I% |
| 6.123 | 14.4227 | 383 | 18.2 | 8620 | 30.3 |
| 6.462 | 13.6668 | 2100 | 100 | 28471 | 100 |
| 7.284 | 12.1266 | 709 | 33.8 | 7155 | 25.1 |
| 12.243 | 7.2231 | 460 | 21.9 | 8465 | 29.7 |
| 12.885 | 6.8646 | 417 | 19.9 | 4461 | 15.7 |
| 13.769 | 6.4261 | 73 | 3.5 | 668 | 2.3 |
| 14.525 | 6.0931 | 85 | 4 | 606 | 2.1 |
| 15.179 | 5.8321 | 94 | 4.5 | 490 | 1.7 |
| 15.967 | 5.5462 | 410 | 19.5 | 4662 | 16.4 |
| 17.366 | 5.1022 | 216 | 10.3 | 3105 | 10.9 |
| 18.286 | 4.8477 | 281 | 13.4 | 3070 | 10.8 |
| 18.646 | 4.7548 | 82 | 3.9 | 1143 | 4 |
| 19.128 | 4.6361 | 130 | 6.2 | 2014 | 7.1 |
| 19.866 | 4.4655 | 127 | 6 | 1935 | 6.8 |
| 20.409 | 4.3479 | 267 | 12.7 | 3111 | 10.9 |
| 21.37 | 4.1544 | 88 | 4.2 | 760 | 2.7 |
| 21.826 | 4.0687 | 155 | 7.4 | 5908 | 20.8 |
| 22.387 | 3.968 | 322 | 15.3 | 6761 | 23.7 |
| 23.126 | 3.8428 | 112 | 5.3 | 1356 | 4.8 |
| 23.63 | 3.7621 | 160 | 7.6 | 2194 | 7.7 |
| 24.333 | 3.6548 | 156 | 7.4 | 5125 | 18 |
| 24.75 | 3.5943 | 257 | 12.2 | 7054 | 24.8 |
| 25.475 | 3.4936 | 152 | 7.2 | 1184 | 4.2 |
| 26.047 | 3.4182 | 128 | 6.1 | 3912 | 13.7 |
| 26.45 | 3.367 | 164 | 7.8 | 5633 | 19.8 |
| 26.727 | 3.3327 | 124 | 5.9 | 6168 | 21.7 |
| 27.305 | 3.2634 | 53 | 2.5 | 95 | 0.3 |
| 28.23 | 3.1586 | 184 | 8.8 | 3448 | 12.1 |
| 30.559 | 2.923 | 52 | 2.5 | 560 | 2 |
| 31.289 | 2.8564 | 78 | 3.7 | 1185 | 4.2 |
| 31.894 | 2.8036 | 80 | 3.8 | 1963 | 6.9 |
| 33.492 | 2.6734 | 125 | 6 | 2788 | 9.8 |
| 36.334 | 2.4705 | 42 | 2 | 718 | 2.5 |
| 37.653 | 2.3869 | 60 | 2.9 | 939 | 3.3 |

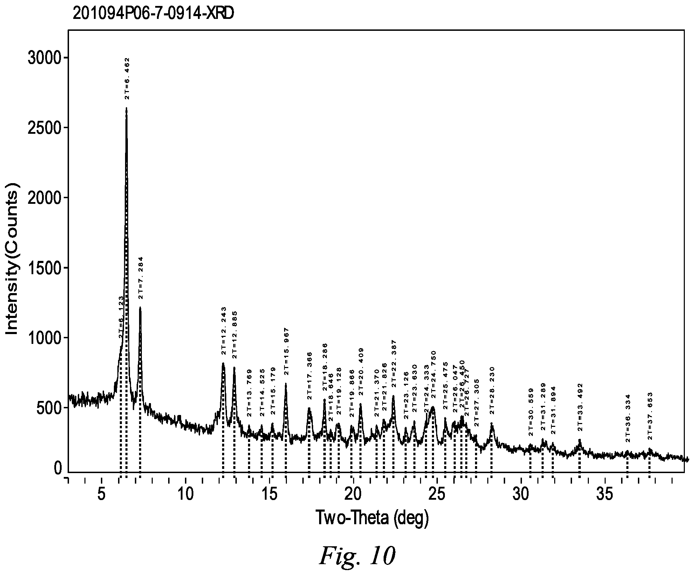

Further, the crystal form II of the hydrobromide of compound A provided by the present application has an X-ray powder diffraction pattern substantially as shown in Figure 10.

The crystal form of the hydrobromide of compound A provided by the present application may exist in the form of a solvate.

The crystal form I of the hydrobromide of compound A provided by the present application may exist in the form of a solvate.

The present application also provides a 2-naphthalenesulfonate of compound A, which is in the form of a crystal (crystal form I of 2-naphthalenesulfonate) and has an X-ray powder diffraction pattern comprising characteristic diffraction peaks at 4.7° ± 0.2°, 9.4° ± 0.2°, 17.2° ± 0.2°, 21.2° ± 0.2° and 23.4° ± 0.2° 2θ, as determined by using Cu-Kα radiation.

Further, the crystal form I of the 2-naphthalenesulfonate of compound A provided by the present application has an X-ray powder diffraction pattern further comprising characteristic diffraction peaks at 6.3° ± 0.2°, 6.8° ± 0.2°, 7.8° ± 0.2°, 13.4° ± 0.2°, 16.5° ± 0.2°, 19.2° ± 0.2° and 20.1° ± 0.2° 2θ.

Further, the crystal form I of the 2-naphthalenesulfonate of compound A provided by the present application has an X-ray powder diffraction pattern further comprising characteristic diffraction peaks at 14.9° ± 0.2°, 15.3° ± 0.2°, 15.7° ± 0.2°, 24.3° ± 0.2°, 25.1° ± 0.2° and 26.1° ± 0.2° 2θ.

Further, the crystal form I of the 2-naphthalenesulfonate of compound A provided by the present application has an X-ray powder diffraction pattern further comprising characteristic diffraction peaks at 11.9° ± 0.2°, 12.3° ± 0.2°, 12.5° ± 0.2°, 13.9° ± 0.2° and 17.5° ± 0.2° 2θ.

Further, the crystal form I of the 2-naphthalenesulfonate of compound A provided by the present application has an X-ray powder diffraction pattern further comprising characteristic diffraction peaks at 18.4° ± 0.2°, 19.7° ± 0.2°, 21.9° ± 0.2°, 23.8° ± 0.2°, 26.1° ± 0.2° and 27.3° ± 0.2° 2θ.

Table 5 shows 2θ values and corresponding intensities in the X-ray powder diffraction pattern of the crystal form I of the 2-naphthalenesulfonate of compound A provided by the present application, wherein the error range of 2θ is ± 0.2°.

**Table 5. 2θ values and corresponding intensities in XRPD pattern of crystal form I of 2-naphthalenesulfonate of compound A**

| **2-Theta** | **d** | **Height** | **1%** | **Area** | **1%** |
|---|---|---|---|---|---|
| 4.7 | 18.7857 | 5380 | 100 | 79756 | 100 |
| 6.299 | 14.0198 | 233 | 4.3 | 2116 | 2.7 |
| 6.753 | 13.0776 | 207 | 3.8 | 748 | 0.9 |
| 7.846 | 11.2585 | 156 | 2.9 | 1187 | 1.5 |
| 9.362 | 9.4384 | 636 | 11.8 | 10368 | 13 |
| 11.863 | 7.454 | 91 | 1.7 | 1324 | 1.7 |
| 12.28 | 7.2016 | 128 | 2.4 | 2679 | 3.4 |
| 12.525 | 7.0614 | 99 | 1.8 | 2675 | 3.4 |
| 13.442 | 6.5816 | 244 | 4.5 | 4697 | 5.9 |
| 13.945 | 6.3456 | 111 | 2.1 | 2523 | 3.2 |
| 14.902 | 5.9398 | 217 | 4 | 3101 | 3.9 |
| 15.325 | 5.7768 | 196 | 3.6 | 5607 | 7 |
| 15.684 | 5.6454 | 123 | 2.3 | 2755 | 3.5 |
| 16.485 | 5.3728 | 309 | 5.7 | 3441 | 4.3 |
| 17.245 | 5.1379 | 562 | 10.4 | 9846 | 12.3 |
| 17.519 | 5.058 | 218 | 4.1 | 4261 | 5.3 |
| 19.188 | 4.6218 | 431 | 8 | 6611 | 8.3 |
| 19.739 | 4.4939 | 94 | 1.7 | 3444 | 4.3 |
| 20.107 | 4.4125 | 160 | 3 | 1828 | 2.3 |
| 20.686 | 4.2903 | 98 | 1.8 | 1686 | 2.1 |
| 21.188 | 4.1898 | 506 | 9.4 | 10750 | 13.5 |
| 21.887 | 4.0576 | 170 | 3.2 | 3076 | 3.9 |
| 22.347 | 3.975 | 78 | 1.4 | 1900 | 2.4 |
| 23.448 | 3.7908 | 543 | 10.1 | 13045 | 16.4 |
| 23.806 | 3.7346 | 305 | 5.7 | 10640 | 13.3 |
| 24.348 | 3.6526 | 304 | 5.7 | 2703 | 3.4 |
| 25.135 | 3.5401 | 235 | 4.4 | 2809 | 3.5 |
| 26.09 | 3.4126 | 193 | 3.6 | 2576 | 3.2 |
| 27.289 | 3.2653 | 192 | 3.6 | 4553 | 5.7 |
| 28.571 | 3.1216 | 77 | 1.4 | 1767 | 2.2 |
| 30.192 | 2.9577 | 88 | 1.6 | 2475 | 3.1 |
| 33.027 | 2.7099 | 67 | 1.2 | 1154 | 1.4 |

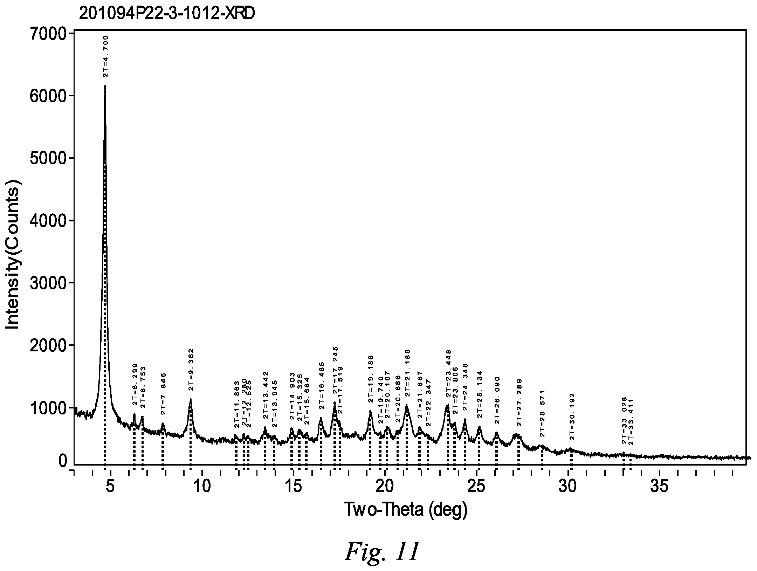

Further, the crystal form I of the 2-naphthalenesulfonate of compound A provided by the present application has an X-ray powder diffraction pattern substantially as shown in Figure 11.

The crystal form I of the 2-naphthalenesulfonate of compound A provided by the present application has a differential scanning calorimetry (DSC) pattern showing an endothermic curve, wherein Tₛₜₐᵣₜ = 143.43°C, Tₚₑₐₖ = 152.36°C and △ H = 46.11 J/g.

Further, the crystal form I of the 2-naphthalenesulfonate of compound A provided by the present application has a melting point of 143.43°C.

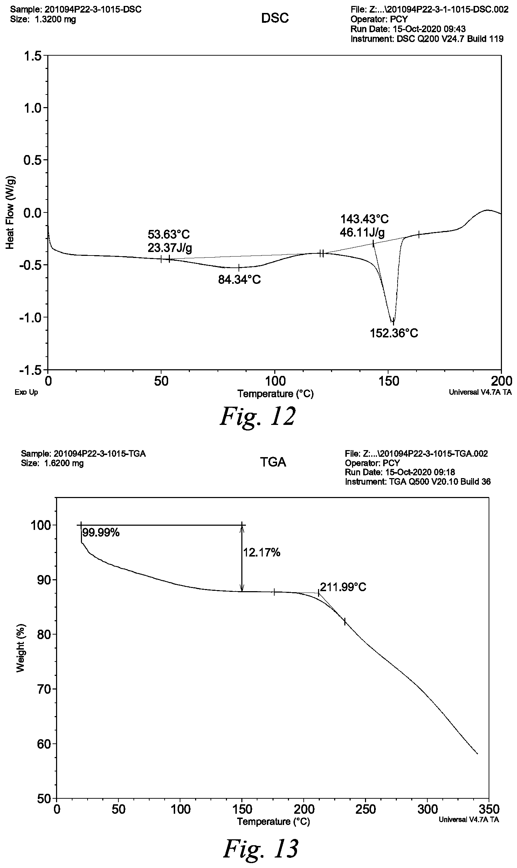

Further, the crystal form I of the 2-naphthalenesulfonate of compound A provided by the present application has a differential scanning calorimetry (DSC) pattern substantially as shown in Figure 12.

The crystal form I of the 2-naphthalenesulfonate of compound A provided by the present application has a thermogravimetric analysis (TGA) curve showing a weight loss of 12.17% below 150°C and showing a decomposition temperature of 211.99°C.

The crystal form I of the 2-naphthalenesulfonate of compound A provided by the present application has a thermogravimetric analysis curve substantially as shown in Figure 13.

The crystal form I of the 2-naphthalenesulfonate of compound A provided by the present application is an anhydride.

The present application also relates to a 2-naphthalenesulfonate of compound A, which is in the form of a crystal (crystal form II of 2-naphthalenesulfonate) and has an X-ray powder diffraction pattern comprising characteristic diffraction peaks at 5.6° ± 0.2°, 11.2° ± 0.2°, 14.1° ± 0.2°, 16.0° ± 0.2°, 22.8° ± 0.2° and 26.8° ± 0.2° 2θ, as determined by using Cu-Kα radiation.

The crystal form II of the 2-naphthalenesulfonate of compound A of the present application has an X-ray powder diffraction pattern further comprising characteristic diffraction peaks at 4.5° ± 0.2°, 6.2° ± 0.2°, 6.8° ± 0.2°, 8.4° ± 0.2°, 10.4° ± 0.2°, 15.3° ± 0.2°, 15.6° ± 0.2°, 19.0° ± 0.2°, 19.6° ± 0.2° and 25.5 ± 0.2° 2θ, as determined by using Cu-Kα radiation.

The crystal form II of the 2-naphthalenesulfonate of compound A of the present application has an X-ray powder diffraction pattern further comprising characteristic diffraction peaks at 12.4° ± 0.2°, 12.7° ± 0.2°, 16.7° ± 0.2°, 17.2° ± 0.2°, 17.5° ± 0.2°, 18.0° ± 0.2°, 20.8° ± 0.2°, 21.8° ± 0.2°, 23.5° ± 0.2° and 24.3° ± 0.2° 2θ, as determined by using Cu-Kα radiation.

Table 6 shows 2θ values and corresponding intensities in the X-ray powder diffraction pattern of the crystal form II of the 2-naphthalenesulfonate of compound A provided by the present application, wherein the error range of 2θ is ± 0.2°.

**Table 6. 2θ values and corresponding intensities in XRPD pattern of crystal form II of 2-naphthalenesulfonate of compound A**

| **2-Theta** | **d** | **Height** | **1%** | **Area** | **1%** |
|---|---|---|---|---|---|
| 4.484 | 19.6908 | 388 | 7 | 3785 | 8.5 |
| 4.735 | 18.6453 | 118 | 2.1 | 1893 | 4.3 |
| 5.6 | 15.7671 | 5548 | 100 | 44273 | 100 |
| 6.185 | 14.2791 | 238 | 4.3 | 1362 | 3.1 |
| 6.814 | 12.9615 | 236 | 4.3 | 1044 | 2.4 |
| 8.359 | 10.5685 | 193 | 3.5 | 990 | 2.2 |
| 9.188 | 9.6172 | 71 | 1.3 | 566 | 1.3 |
| 10.404 | 8.4958 | 268 | 4.8 | 1809 | 4.1 |
| 11.202 | 7.8921 | 2294 | 41.3 | 19034 | 43 |
| 11.719 | 7.5452 | 71 | 1.3 | 782 | 1.8 |
| 12.427 | 7.117 | 141 | 2.5 | 1541 | 3.5 |
| 12.688 | 6.9712 | 190 | 3.4 | 2376 | 5.4 |
| 13.602 | 6.5045 | 106 | 1.9 | 718 | 1.6 |
| 14.142 | 6.2575 | 440 | 7.9 | 2968 | 6.7 |
| 14.799 | 5.9812 | 108 | 1.9 | 373 | 0.8 |
| 15 | 5.9013 | 156 | 2.8 | 1032 | 2.3 |
| 15.342 | 5.7706 | 458 | 8.3 | 5650 | 12.8 |
| 15.585 | 5.6813 | 437 | 7.9 | 5702 | 12.9 |
| 15.965 | 5.5469 | 639 | 11.5 | 4859 | 11 |
| 16.327 | 5.4245 | 99 | 1.8 | 683 | 1.5 |
| 16.745 | 5.2901 | 305 | 5.5 | 2391 | 5.4 |
| 17.241 | 5.1389 | 244 | 4.4 | 3004 | 6.8 |
| 17.503 | 5.0626 | 431 | 7.8 | 5022 | 11.3 |
| 18.026 | 4.917 | 275 | 5 | 2996 | 6.8 |
| 18.788 | 4.7191 | 84 | 1.5 | 1290 | 2.9 |
| 19.01 | 4.6646 | 409 | 7.4 | 5084 | 11.5 |
| 19.604 | 4.5245 | 404 | 7.3 | 3186 | 7.2 |
| 20.788 | 4.2695 | 260 | 4.7 | 5459 | 12.3 |
| 21.291 | 4.1696 | 118 | 2.1 | 1530 | 3.5 |
| 21.646 | 4.1021 | 206 | 3.7 | 5770 | 13 |
| 21.81 | 4.0717 | 268 | 4.8 | 6848 | 15.5 |
| 22.068 | 4.0246 | 236 | 4.3 | 3141 | 7.1 |
| 22.789 | 3.8989 | 591 | 10.7 | 11207 | 25.3 |
| 23.468 | 3.7876 | 286 | 5.2 | 5837 | 13.2 |
| 24.329 | 3.6555 | 431 | 7.8 | 7140 | 16.1 |
| 24.57 | 3.6202 | 242 | 4.4 | 5272 | 11.9 |
| 25.05 | 3.5518 | 131 | 2.4 | 3610 | 8.2 |
| 25.45 | 3.4969 | 429 | 7.7 | 7151 | 16.2 |
| 26.832 | 3.3199 | 562 | 10.1 | 7830 | 17.7 |
| 27.35 | 3.2582 | 87 | 1.6 | 1186 | 2.7 |
| 27.758 | 3.2113 | 73 | 1.3 | 936 | 2.1 |
| 28.248 | 3.1566 | 91 | 1.6 | 2224 | 5 |
| 28.468 | 3.1327 | 66 | 1.2 | 2144 | 4.8 |
| 30.233 | 2.9538 | 137 | 2.5 | 2240 | 5.1 |
| 31.427 | 2.8442 | 48 | 0.9 | 347 | 0.8 |
| 32.248 | 2.7736 | 78 | 1.4 | 471 | 1.1 |
| 34.855 | 2.5719 | 66 | 1.2 | 1008 | 2.3 |

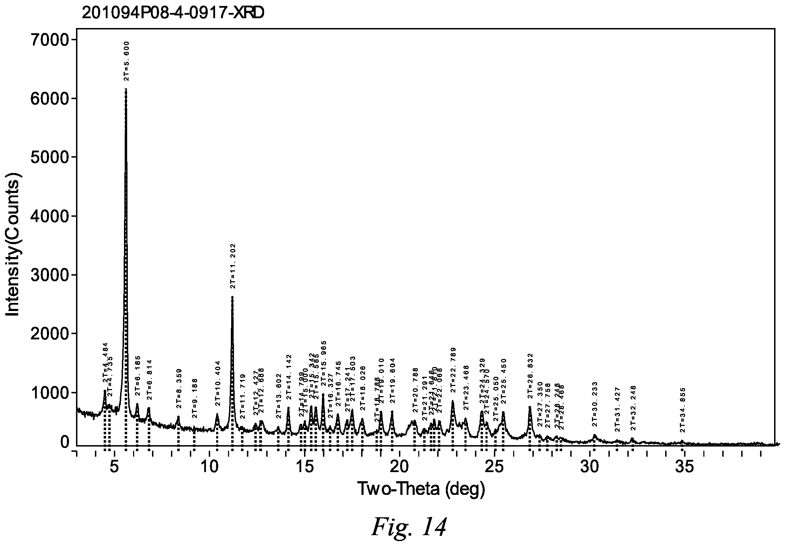

The crystal form II of the 2-naphthalenesulfonate of compound A of the present application has an X-ray powder diffraction pattern substantially as shown in Figure 14.

The salt or crystal form of the present application accounts for approximately 5 wt% to approximately 100 wt% of a bulk drug.

In some embodiments, the salt or crystal form of the present application accounts for approximately 10 wt% to approximately 100 wt% of the bulk drug.

In some embodiments, the salt or crystal form of the present application accounts for approximately 15 wt% to approximately 100 wt% of the bulk drug.

In some embodiments, the salt or crystal form of the present application accounts for approximately 20 wt% to approximately 100 wt% of the bulk drug.

In some embodiments, the salt or crystal form of the present application accounts for approximately 25 wt% to approximately 100 wt% of the bulk drug.

In some embodiments, the salt or crystal form of the present application accounts for approximately 30 wt% to approximately 100 wt% of the bulk drug.

In some embodiments, the salt or crystal form of the present application accounts for approximately 35 wt% to approximately 100 wt% of the bulk drug.

In some embodiments, the salt or crystal form of the present application accounts for approximately 40 wt% to approximately 100 wt% of the bulk drug.

In some embodiments, the salt or crystal form of the present application accounts for approximately 45 wt% to approximately 100 wt% of the bulk drug.

In some embodiments, the salt or crystal form of the present application accounts for approximately 50 wt% to approximately 100 wt% of the bulk drug.

In some embodiments, the salt or crystal form of the present application accounts for approximately 55 wt% to approximately 100 wt% of the bulk drug.

In some embodiments, the salt or crystal form of the present application accounts for approximately 60 wt% to approximately 100 wt% of the bulk drug.

In some embodiments, the salt or crystal form of the present application accounts for approximately 65 wt% to approximately 100 wt% of the bulk drug.

In some embodiments, the salt or crystal form of the present application accounts for approximately 70 wt% to approximately 100 wt% of the bulk drug.

In some embodiments, the salt or crystal form of the present application accounts for approximately 75 wt% to approximately 100 wt% of the bulk drug.

In some embodiments, the salt or crystal form of the present application accounts for approximately 80 wt% to approximately 100 wt% of the bulk drug.

In some embodiments, the salt or crystal form of the present application accounts for approximately 85 wt% to approximately 100 wt% of the bulk drug.

In some embodiments, the salt or crystal form of the present application accounts for approximately 90 wt% to approximately 100 wt% of the bulk drug.

In some embodiments, the salt or crystal form of the present application accounts for approximately 95 wt% to approximately 100 wt% of the bulk drug.

In some embodiments, the salt or crystal form of the present application accounts for approximately 98 wt% to approximately 100 wt% of the bulk drug.

In some embodiments, the salt or crystal form of the present application accounts for approximately 99 wt% to approximately 100 wt% of the bulk drug.

In some embodiments, the salt or crystal form of the present application substantially accounts for 100 wt% of the bulk drug, that is, the bulk drug is substantially a pure phase salt or crystal.

It can be understood that, as is well known in the field of differential scanning calorimetry (DSC), a melting peak height of a DSC curve depends on many factors related to sample preparation and geometric shapes of instruments, and a peak position is relatively insensitive to experiment details. Therefore, in some embodiments, the crystallized compounds of the present application have DSC patterns comprising characteristic peak positions, wherein the DSC patterns have substantially the same properties as those provided in the drawings of the present application, with an error tolerance of ± 3°C.

The present application also relates to a pharmaceutical composition comprising a therapeutically effective amount of the salt of compound A, or the hydrate, solvate or crystal form of the salt thereof according to the present application, and a pharmaceutically acceptable carrier or excipient.

The present application also relates to the use of the salt of compound A, or the hydrate, solvate or crystal form of the salt thereof, or the pharmaceutical composition, in the preparation of a medicament for preventing and/or treating influenza.

The present application also relates to a method for treating and/or preventing influenza, comprising administering to a subject in need thereof a therapeutically and/or prophylactically effective amount of the salt of compound A, or the hydrate, solvate or crystal form of the salt thereof, or the pharmaceutical composition according to the present application.

The present application also relates to the salt of compound A, or the hydrate, solvate or crystal form of the salt thereof, or the pharmaceutical composition, for use in the treatment and/or prevention of influenza.

The present application further provides a composition for treating and/or preventing influenza, comprising the salt of compound A, or the hydrate, solvate or crystal form of the salt thereof, or the pharmaceutical composition.

Unless stated to the contrary, the terms used in the description and claims have the following meanings.

The carbon, hydrogen, oxygen, sulphur, nitrogen and halogen involved in the groups and compounds of the present application all comprise isotopes thereof, and are optionally further substituted with one or more of the corresponding isotopes thereof, wherein the isotopes of carbon comprise ¹²C, ¹³C and ^{1j}; the isotopes of hydrogen comprise protium (H), deuterium (D, also known as heavy hydrogen), and tritium (T, also known as superheavy hydrogen); the isotopes of oxygen comprise ¹⁶O, ¹⁷O and ¹⁸O; the isotopes of sulphur comprise ³²S, ³³S, ³⁴S and ³⁶S; the isotopes of nitrogen comprise ¹⁴N and ¹⁵N; the isotope of fluorine comprises ¹⁹F; the isotopes of chlorine comprise ³⁵Cl and ³⁷Cl; and the isotopes of bromine comprise ⁷⁹Br and ⁸¹Br.

The "effective amount" means an amount of a compound that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "IC₅₀" refers to the half maximal inhibitory concentration, i.e., a concentration where half of the maximum inhibitory effect is achieved.

As used in the present application, the expression "substantially as shown in figure..." for defining the figures is intended to mean that, in view of acceptable deviations in the art, a person skilled in the art would consider that the figures are the same as the reference figures. Such deviations may be caused by known factors in the art related to instruments, operating conditions, human factors, etc. For example, a person skilled in the art would have appreciated that an endothermic start temperature and an endothermic peak temperature measured by differential scanning calorimetry (DSC) can vary significantly with experiments. In some embodiments, it is considered that two patterns are substantially identical when the change in the positions of characteristic peaks of the two patterns does not exceed ± 5%, ± 4%, ± 3%, ± 2% or ± 1%. For example, it would have readily occurred to a person skilled in the art to identify whether two X-ray diffraction patterns or two DSC patterns are substantially identical. In some embodiments, it is considered that X-ray diffraction patterns are substantially identical when the change in the 2θ angle of characteristic peaks of the two X-ray diffraction patterns does not exceed ± 0.3°, ± 0.2° or ± 0.1°.

As used in the present application, the term "approximately" should be understood to be within a range of normal tolerance in the art, for example, "approximately" can be understood to be within ± 10%, ± 9%, ± 8%, ± 7%, ± 6%, ± 5%, ± 4%, ± 3%, ± 2%, ± 1%, ± 0.5%, ± 0.1%, ± 0.05% or ± 0.01% of the value. Unless otherwise obvious from the context, all numeric values provided by the present application are modified with the term "approximately".

As used in the present application, the term "pharmaceutically acceptable carrier or excipient" refers to a diluent, adjunct or vehicle that is administered with a therapeutic agent and is, within the scope of sound medical judgment, suitable for use in contact with the tissues of human beings and animals without undue toxicity, irritation, allergic response, or other problems or complications, commensurate with a reasonable benefit/risk ratio.

According to the salt of compound A, or the hydrate, solvate or crystal form of the salt thereof of the present application, the specific dosage and use method for different patients depend on many factors, including the age, weight, gender, natural health status and nutritional status of a patient, the active intensity, taking time and metabolic rate of the compound, the severity of a disorder, and the subjective judgment of a diagnosing and treating physician. A dosage of 0.01-1000 mg/kg body weight/day is preferably used here.

The structure of the crystal form of the present application can be analysed by using various analytical techniques known to a person skilled in the art, including but not limited to X-ray powder diffraction (XRD), differential scanning calorimetry (DSC) and/or thermogravimetry (TG).

Thermogravimetric analysis (TGA) is also called as thermogravimetry (TG).

### Brief Description of the Drawings

Figure 1 shows the X-ray powder diffraction pattern of crystal form I of hemi-1,5-naphthalene disulfonate of compound A.
Figure 2 shows the DSC pattern of crystal form I of hemi-1,5-naphthalene disulfonate of compound A.
Figure 3 shows the TGA curve of crystal form I of hemi-1,5-naphthalene disulfonate of compound A.
Figure 4 shows the X-ray powder diffraction pattern of crystal form I of hydrochloride of compound A.
Figure 5 shows the DSC pattern of crystal form I of hydrochloride of compound A.
Figure 6 shows the TGA curve of crystal form I of hydrochloride of compound A.
Figure 7 shows the X-ray powder diffraction pattern of crystal form I of hydrobromide of compound A.
Figure 8 shows the DSC pattern of crystal form I of hydrobromide of compound A.
Figure 9 shows the TGA curve of crystal form I of hydrobromide of compound A.
Figure 10 shows the X-ray powder diffraction pattern of crystal form II of hydrobromide of compound A.
Figure 11 shows the X-ray powder diffraction pattern of crystal form I of 2-naphthalenesulfonate of compound A.
Figure 12 shows the DSC pattern of crystal form I of 2-naphthalenesulfonate of compound A.
Figure 13 shows the TGA curve of crystal form I of 2-naphthalenesulfonate of compound A.
Figure 14 shows the X-ray powder diffraction pattern of crystal form II of 2-naphthalenesulfonate of compound A.

### Detailed Description of Embodiments

The implementation process and beneficial effects of the present application are described in detail below through specific examples, which are intended to help readers better understand the essence and characteristics of the present application and are not intended to limit the scope of implementation of the present application.

The structure of the compound is determined by nuclear magnetic resonance (NMR) and/or mass spectrometry (MS).

NMR is determined with Bruker ADVANCE III 400; the solvent for determination is deuterated dimethyl sulfoxide (DMSO-*d₆*), deuterated chloroform (CDCl₃) and deuterated methanol (CD₃OD); and the internal standard is tetramethylsilane (TMS).

MS is determined with Agilent 6120B (ESI).

HPLC is determined with Agilent 1260DAD high pressure liquid chromatograph (Zorba × SB-C18 100 × 4.6 mm).

For the column chromatography, Yantai Huanghai silica gel of 200-300 mesh silica gel is generally used as a carrier.

### Instrument:

| **X-ray powder diffractometer (XRPD) and hot-stage XRPD** | | | |
|---|---|---|---|
| Instrument | Model | Bruker D8 Advance Diffractometer | |
| | No. | LY-01-034 | |
| | Technical indicator | Kα radiation (40 KV, 40 mA) with a copper target wavelength of 1.54 Å, a θ-2θ goniometer, nickel filtration and a Lynxeye detector | |
| | Acquisition software | Diffrac Plus XRD Commander | |
| | Calibration material | Corundum (Al₂O₃) | |
| | Analysis software | MDI Jade | |
| Accessory | Non-reflective sample plate | Specification | 24.6 mm diameter × 1.0 mm thickness |
| | | Manufacturer | MTI corporation |

| **Differential scanning calorimeter (DSC)** | | | |
|---|---|---|---|
| Instrument | Model | METTLER TOLEDO DSC 3 | |
| | No. | LY-01-167 | |
| | Control software | STARe software | |
| | Analysis software | STARe software | |
| | Sample tray | Aluminium crucible (with a cover and with perforation) | |
| Parameter | Sample size | 0.5 mg - 5 mg | |
| | Protective gas | Nitrogen gas | |
| | Gas flow rate | 50 mL/min | |
| | Detection method | Segment 1 | |
| | | Start temp 25°C | |
| | | End temp 350 | |
| | | Heating rate 10.0 k/min | |

| **Thermal gravimetric analyser (TGA)** | | | |
|---|---|---|---|
| Instrument | Model | METTLER TOLEDO TGA/DSC 3⁺ | |
| | No. | LY-01-166 | |
| | Control software | STARe software | |
| | Analysis software | STARe software | |
| | Sample tray | 70 µL ceramic crucible | |
| Parameter | Sample size | 1 mg - 10 mg | |
| | Protective gas | Nitrogen gas | |
| | Gas flow rate | 50 mL/min | |
| | Detection method | Segment 1 (MaxRes) | |
| | | Start temp 25.0°C | |
| | | End temp 120.0°C | |
| | | Heating rate 10.0 k/min | |
| | | Segment 2 | |
| | | Start temp 120.0°C | |
| | | End temp 350.0°C | |
| | | Heating rate 10.0 k/min | |

Unless otherwise specified in the examples, a solution refers to an aqueous solution.

Unless otherwise specified in the examples, a reaction is performed at room temperature,
and room temperature refers to 10°C-30°C.

### Description of abbreviations:

DDQ: 2,3-dichloro-5,6-dicyano-1,4-benzoquinone;
Pd(dppf)Cl₂: 1,1'-bis(diphenylphosphino)ferrocene dichloropalladium (II);
EA: ethyl acetate;
PE: petroleum ether;
THF: tetrahydrofuran;
DEAD: diethyl azodicarboxylate;
DMF: N, N-dimethylformamide;
HATU: 2-(7-azobenzotriazole)-N,N,N' ,N' -tetramethyluronium hexafluorophosphate;
DIEA: N,N-diisopropylethylamine;
SBE-β-CD: sulfobutyl ether β-cyclodextrin;
DMA: dimethylacetamide;
MC: methylcellulose;
DMSO: dimethyl sulfoxide.

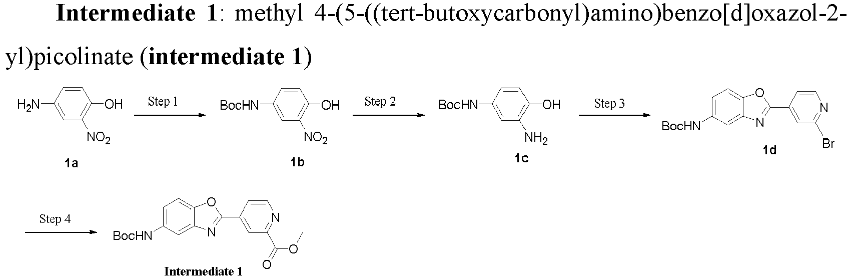

### Step 1: preparation of tert-butyl-(4-hydroxy-3-nitrophenyl)carbamate (1b)

Tetrahydrofuran (50 mL) and di-tert-butyl dicarbonate (10.6 g, 48.7 mmol) were successively added to known compound **1a** (5.0 g, 32.5 mmol). After the addition, the mixture was warmed to 70°C, reacted for 16 h and concentrated under reduced pressure to remove tetrahydrofuran. The resulting mixture was slurried with petroleum ether (100 mL) for 1 h and then filtered. The filter cake was collected and dried to obtain compound **1b** (6.1 g, 74%).

¹H NMR (400 MHz, CD₃OD) δ 8.25 (d, 1H), 7.56 (d, 1H), 7.06 (d, 1H) ,1.52 (s, 9H).

LC-MS (ESI): m/z =255.1[M+H]⁺.

### Step 2: preparation of tert-butyl-(3-amino-4-hydroxyphenyl)carbamate (1c)

At room temperature, compound **1b** (6.1 g, 24.0 mmol) was dissolved in anhydrous methanol (60 mL). Pd/C (2.1 g, with Pd content of 10% and water content of 50%) was added. Hydrogen was introduced. The mixture was warmed to 45°C and reacted for 5 h. After filtration, the filtrate was concentrated to obtain compound **1c** (4.3 g, 80%).

LC-MS (ESI): m/z =225.1[M+H]⁺.

### Step 3: preparation of tert-butyl (2-(2-bromopyridin-4-yl)-2,3-dihydrobenzo[d]oxazol-5-yl)carbamate (1d)

Compound **1c** (4.3 g, 19.2 mmol) was dissolved in methanol (50 mL). 2-bromopyridine-4-carboxaldehyde (3.6 g, 19.2 mmol) was added. The mixture was warmed to 70°C and stirred for 15 h. The reaction solution was cooled to room temperature and concentrated under reduced pressure to remove methanol. Then dichloromethane (200 mL) and DDQ (5.3 g, 23.0 mmol) were successively added to the residue. After the addition, the mixture was stirred for 2 h at room temperature, and a saturated aqueous sodium carbonate solution (100 mL) was added. The resulting solution was stirred for 10 min and filtered. The filtrate was extracted with dichloromethane (200 mL × 2). The combined organic phase was washed with water (100 mL), dried over anhydrous sodium sulphate and filtered. The filtrate was concentrated under reduced pressure, and then the residue was separated and purified by column chromatography (eluent: EA/PE = 10% - 50%) to obtain compound **1d** (4.1 g, 54%).

LC-MS (ESI): m/z =392.1[M+H]⁺.

### Step 4: preparation of methyl 4-(5-((tert-butoxycarbonyl)amino)benzo[d]oxazol-2-yl)picolinate (intermediate 1)

Methanol (25 mL), dichloromethane (25 mL), Pd(dppf)Cl₂ (804.0 mg, 1.1 mmol) and triethylamine (4.24 g, 42.0 mmol) were successively added to compound **1d** (4.1 g, 10.5 mmol). Carbon monoxide was introduced; and then the reaction solution was warmed to 120°C, stirred for 14 h, cooled to room temperature and then filtered. The filtrate was concentrated under reduced pressure, and then the residue was separated and purified by column chromatography (eluent: EA/PE = 10% - 50%) to obtain **intermediate 1** (3.5 g, 90%).

¹H NMR (400 MHz, CDCl₃) δ 8.95 (d, 1H), 8.89 (d, 1H), 8.26 (d, 1H), 7.86 (s, 1H), 7.54-7.47 (m, 2H), 6.67 (s, 1H), 4.08 (s, 3H), 1.55 (s, 9H).

LC-MS (ESI): m/z =370.1[M+H]⁺.

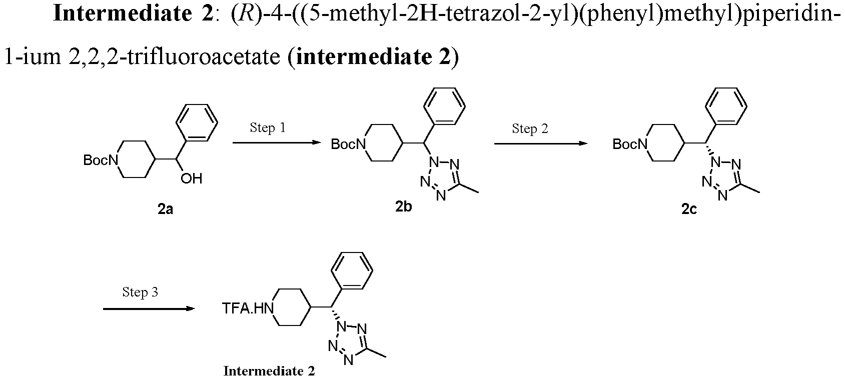

### Step 1: preparation of tert-butyl 4-((5-methyl-2H-tetrazol-2-yl)(phenyl)methyl)piperidine-1-carboxylate (2b)

At room temperature, compound **2a** (580 mg, 2.0 mmol), 5-methyltetrazole (185 mg, 2.2 mmol) and triphenylphosphine (787 mg, 3.0 mmol) were dissolved in anhydrous THF (20 mL). The mixture was cooled to 0°C under nitrogen protection, and then DEAD (520 mg, 3.0 mmol) was added dropwise. The mixture was allowed to naturally warm to room temperature, reacted overnight, concentrated under reduced pressure and subjected to column chromatography to obtain compound **2b** (440 mg, 61.0%).

LC-MS (ESI): m/z =358.3[M+H]⁺.

### Step 2: preparation of (R)-tert-butyl 4-((5-methyl-2H-tetrazol-2-yl)(phenyl)methyl)piperidine-1-carboxylate (2c)

Compound **2b** was resolved by chiral HPLC to obtain compound **2c** (tR = 1.78 min, 200 mg, 45.5%).

Resolution conditions were as follows:
instrument: MG II preparative SFC (SFC-1); column type: ChiralCel OJ, 250×30 mm I.D., 5 µm; mobile phase: A: CO₂, B: ethanol; gradient: B 15%; flow rate: 60 mL /min; back pressure: 100 bar; column temperature: 38°C; column length: 220 nm; time cycle: about 5 min; sample preparation: 0.44 g of compound **2b** was dissolved in a mixed solvent (4 mL) of dichloromethane and methanol; sample injection: 2 mL/injection.

### Step 3: preparation of (A)-4-((5-methyl-2H-tetrazol-2-yl)(phenyl)methyl)piperidin-1-ium 2,2,2-trifluoroacetate (intermediate 2)

At room temperature, compound **2c** (200 mg, 0.55 mmol) was dissolved in dichloromethane (10 mL). Trifluoroacetic acid (2.5 mL) was added dropwise, and the mixture was stirred for another 2 h. The reaction solution was subjected to rotary evaporation to obtain a crude of **intermediate 2** (300 mg), which was directly used in the next reaction without purification.

LC-MS (ESI): m/z =258.2[M+H]⁺.

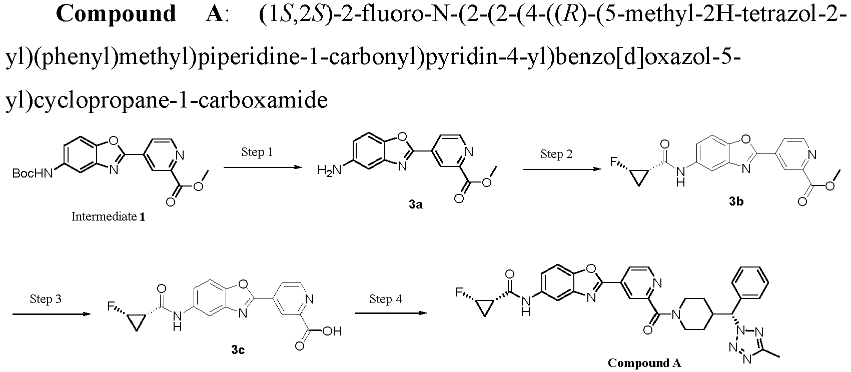

### Step 1: preparation of methyl-4-(5-aminobenzo[d]oxazol-2-yl)picolinate (3a)

**Intermediate 1** (600.0 mg, 1.62 mmol) was dissolved in dichloromethane (5 mL), and trifluoroacetic acid (2 mL) was added. After the addition, the mixture was stirred for 2 h at room temperature, adjusted to pH = 8-9 with a saturated aqueous sodium carbonate solution and extracted with dichloromethane (50 mL × 2). The organic phases were combined, dried and filtered. The filtrate was concentrated to obtain compound **3a** (396.0 mg, 90%).

LC-MS (ESI): m/z =270.1[M+H]⁺.

### Step 2: preparation of methyl-4-(5-((1S,2S)-2-fluorocyclopropane-1-carboxamido)benzo[d]oxazol-2-yl)picolinate (3b)

DMF (50 mL), (15,2S)-2-fluorocyclopropanecarboxylic acid (425 mg, 4.1 mmol), HATU (2.1 g, 5.58 mmol) and DIEA (1.44 g, 11.16 mmol) were successively added to compound **3a** (1.0 g, 3.71 mmol), and the mixture was stirred at room temperature for 5 h. The reaction was quenched by adding water, extracted 3 times with ethyl acetate and washed twice with saturated brine. The organic phase was dried and concentrated, and the residue was separated and purified by silica gel column chromatography (eluent: EA/PE = 1/2) to obtain compound **3b** (1.1 g, 83.4%).

LC-MS (ESI): m/z =356.3 [M+H]⁺.

### Step 3: preparation of 4-(5-((1S,2S)-2-fluorocyclopropane-1-carboxamido)benzo[d]oxazol-2-yl)picolinic acid (3c)

At room temperature, compound **3b** (1 g, 3.1 mmol) was dissolved in methanol (15 mL), and lithium hydroxide (700 mg) was dissolved in 20 mL of pure water. An aqueous solution of lithium hydroxide was added to the reaction solution. The mixture was stirred at 40°C for 0.5 h and then adjusted to pH = 6-7 with 2N hydrochloric acid. A large amount of solid was precipitated out, filtered by suction and washed with water (10 mL × 3). The filter cake was dried at 50°C to obtain compound **3c** (1.0 g, 94.6%).

LC-MS (ESI): m/z =342.1 [M+H]⁺.

### Step 4: preparation of (1S,2S)-2-fluoro-N-(2-(2-(4-((R)-(5-methyl-2H-tetrazol-2-yl)(phenyl)methyl)piperidine-1-carbonyl)pyridin-4-yl)benzo[d]oxazol-5-yl)cyclopropane-1-carboxamide (compound A)

At room temperature, compound **3c** (170 mg, 0.5 mmol) and DIPEA (130 mg, 1.0 mmol) were dissolved in DMF (5 mL), and then HATU (230 mg, 0.6 mmol) was added. The mixture was stirred for 3 min, and then intermediate **2** (300 mg, approximately 0.55 mmol) was added. The mixture was reacted for another 30 min at room temperature. 30 mL of water was added, and the reaction solution was extracted with ethyl acetate (30 mL × 3). The organic phases were combined, washed with saturated sodium chloride (30 mL × 1), dried over anhydrous sodium sulphate and concentrated under reduced pressure, and then the residue was subjected to column chromatography (DCM : MeOH = 30 : 1-15 : 1) to obtain compound **A** (130 mg, 44.8%), which was an amorphous form as identified by XPRD.

LC-MS (ESI): m/z =581.3[M+H]⁺.

¹H NMR (400 MHz, CDCl₃) δ 8.74-8.71(m, 1H), 8.31(s, 1H), 8.12-8.10(m, 1H), 8.01(s, 1H), 7.90-7.87(m, 1H), 7.56-7.51(m,4H), 7.41-7.31(m, 3H), 5.55-5.52(m, 1H), 4.93-4.75(m, 2H), 3.91-3.88(m, 1H), 3.20-3.11(m, 1H), 2.91-2.80(m, 2H), 2.56-2.50(m, 3H), 1.94-1.84(m, 2H), 1.61-1.23(m, 5H).

### Example 1 Preparation of hemi-1,5-naphthalene disulfonate of compound A

At room temperature, 200 mg of the amorphous compound **A** was dissolved in 16.60 mL of isopropanol to obtain solution 1a; 150.94 mg of 1,5-naphthalene disulfonic acid was dissolved in 1.80 mL of isopropanol to obtain solution 1b; at room temperature with stirring, solution 1b was added dropwise to solution 1a to obtain solution 1c; solution 1c was continuously stirred, and a solid was precipitated immediately to obtain a suspension; the suspension was stirred overnight and then centrifuged; and the resulting solid was dried under vacuum at room temperature to obtain the hemi-1,5-naphthalene disulfonate of compound **A.** The compound was identified as a crystalline form by XRPD and named as crystal form I of hemi-1,5-naphthalene disulfonate of compound **A.**

¹H NMR (400 MHz, DMSO-*d6*) δ 10.48 (s, 1H), 8.92 - 8.85 (m, 1H), 8.84 - 8.77 (m, 1H), 8.25 (d, 1H), 8.18 - 8.11 (m, 2H), 7.95 (dd, 1H), 7.80 (d, 1H), 7.69 - 7.53 (m, 3H), 7.48 - 7.30 (m, 4H), 5.93 (dd, 1H), 5.07 - 4.82 m, 1H), 4.50 (d, 1H), 3.75 - 3.68 (m, 1H), 3.08 (t, 1H), 2.86 (d, 2H), 2.46 (d, 3H), 2.10 - 2.00 (m, 1H), 1.75 - 1.60 (m, 1H), 1.46 -1.13 (m, 5H).

The crystal form I of hemi-1,5-naphthalene disulfonate of compound **A** obtained in example 1 is a crystalline form as identified by XRPD. Table 1 shows the XRPD peak list; Figure 1 shows the XRPD pattern; and Figure 3 shows the TGA pattern exhibiting a weight loss of 4.017% below 150°C and exhibiting a decomposition temperature of approximately 213.86°C. Figure 2 shows the DSC pattern, with a melting point of approximately 188.78°C.

### Example 2: Preparation of hydrochloride of compound A

At room temperature, 100 mg of the amorphous compound **A** was ultrasonically dissolved in 2.40 mL of acetone to obtain a clear solution, i.e., solution 2a; 20.17 mg of hydrochloric acid was dissolved in 2.66 mL of acetone to obtain solution 2b; at room temperature with stirring, solution 2b was added dropwise to solution 2a to obtain solution 2c; solution 2c was continuously stirred overnight, and then a solid was precipitated to obtain a suspension; the suspension was centrifuged; and the resulting solid was dried under vacuum at room temperature to obtain the hydrochloride of compound **A.** The compound was identified as a crystalline form by XRPD and named as crystal form I of hydrochloride of compound **A.**

¹H NMR (400 MHz, DMSO-*d6*) δ 10.54 (s, 1H), 8.80 (t, 1H), 8.25 (d, 1H), 8.19 - 8.10 (m, 2H), 7.80 (d, 1H), 7.67 - 7.53 (m, 3H), 7.47 - 7.28 (m, 3H), 5.93 (dd, 1H), 5.10 - 4.85 (m, 1H), 4.50 (d, 1H), 3.72 (d, 1H), 3.08 (t, 1H), 2.89 - 2.79 (m, 2H), 2.45 (d, 3H), 2.07 - 1.93 (m, 1H), 1.75 - 1.60 (m, 1H), 1.44 - 1.10 (m, 5H).

The crystal form I of hydrochloride of compound **A** obtained in example 2 is a crystalline form as identified by XRPD. Table 2 shows the XRPD peak list; Figure 4 shows the XRPD pattern; and Figure 6 shows the TGA pattern exhibiting a weight loss of 3.202% below 150°C and exhibiting a decomposition temperature of approximately 171.90°C. Figure 5 shows the DSC pattern, with a melting point of approximately 118.97°C.

### Example 3: Preparation of hydrobromide of compound A

### Method I:

At room temperature, 100 mg of the amorphous compound **A** was ultrasonically dissolved in 2.40 mL of acetone to obtain a clear solution, i.e., solution 3a; 40.50 mg of hydrobromic acid was dissolved in 2.33 mL of acetone to obtain solution 3b; at room temperature with stirring, solution 3b was added dropwise to solution 3a to obtain solution 3c; solution 3c was continuously stirred for 1 h, and then a solid was precipitated to obtain a suspension; the suspension was stirred and then centrifuged; and the resulting solid was dried under vacuum at room temperature to obtain the hydrobromide of compound **A.** The compound was identified as a crystalline form by XRPD and named as crystal form I of hydrobromide of compound **A.**

### Method II:

At room temperature, 30 mg of the amorphous compound **A** was ultrasonically dissolved in 0.2 mL of acetone to obtain a clear solution, i.e., solution 3a-1; 12.32 mg of hydrobromic acid was dissolved in 0.7 mL of acetone to obtain solution 3b-1; at room temperature with stirring, solution 3b-1 was added dropwise to solution 3a-1 to obtain solution 3c-1; solution 3c-1 was continuously stirred for a few minutes, and then a solid was precipitated to obtain a suspension; the suspension was stirred overnight and then centrifuged; and the resulting solid was dried under vacuum at room temperature to obtain the hydrobromide of compound **A.** The compound was identified as a crystalline form by XRPD and named as crystal form II of hydrobromide of compound **A.**

¹H NMR (400 MHz, DMSO-*d6*) δ 10.50 (s, 1H), 8.81 (t, 1H), 8.25 (d, 1H), 8.19 - 8.10 (m, 2H), 7.80 (d, 1H), 7.69 - 7.51 (m, 3H), 7.46 - 7.27 (m, 3H), 5.93 (dd, 1H), 5.10 - 4.86 (m, 1H), 4.50 (d, 1H), 3.72 (d, 1H), 3.08 (t, 1H), 2.86 (d, 2H), 2.45 (d, 3H), 2.08 - 1.98 (m, 1H), 1.75 - 1.61 (m, 1H), 1.45 - 1.10 (m, 5H).

The crystal form I of hydrobromide of compound **A** obtained in example 3 is a crystalline form as identified by XRPD. Table 3 shows the XRPD peak list; Figure 7 shows the XRPD pattern; and Figure 9 shows the TGA pattern exhibiting a weight loss of 3.584% below 100°C and a weight loss of 7.033% between 100°C-150°C and exhibiting a decomposition temperature of 185.29°C. Figure 8 shows the DSC pattern, with a melting point of approximately 179.68°C.

The crystal form II of hydrobromide of compound **A** obtained in example 3 is a crystalline form as identified by XRPD. Table 4 shows the XRPD peak list; and Figure 10 shows the XRPD pattern.

### Example 4: Preparation of 2-naphthalenesulfonate of compound A

### Method I:

At room temperature, approximately 100 mg of the amorphous compound A was dissolved in 0.67 mL of tetrahydrofuran to obtain solution 4a; 40.27 mg of 2-naphthalenesulfonic acid was dissolved in 2.16 mL of tetrahydrofuran to obtain solution 4b; at room temperature with stirring, solution 4b was added dropwise to solution 4a to obtain solution 4c; solution 4c was continuously stirred for a few minutes, and then a solid was precipitated to obtain a suspension; the suspension was stirred overnight and then centrifuged; and the resulting solid was dried under vacuum at room temperature to obtain the 2-naphthalenesulfonate of compound A. The compound was identified as a crystalline form by XRPD and named as crystal form I of 2-naphthalenesulfonate of compound A.

### Method II:

At room temperature, 30 mg of the amorphous compound A was ultrasonically dissolved in 0.20 mL of 1,4-dioxane to obtain a clear solution, i.e., solution 4d; 12.0 mg of 2-naphthalenesulfonic acid was ultrasonically dissolved in 1.00 mL of 1,4-dioxane to obtain a clear solution, i.e., solution 4e; at room temperature with stirring, solution 4e was added dropwise to solution 4d to obtain solution 4f; solution 4f was continuously stirred, and then a solid was precipitated to obtain a suspension; the suspension was stirred overnight and then centrifuged; and the resulting solid was dried under vacuum at room temperature to obtain the 2-naphthalenesulfonate of compound A. The compound was identified as a crystalline form by XRPD and named as crystal form I of 2-naphthalenesulfonate of compound A.

### Method III:

At room temperature, 30 mg of the amorphous compound A was ultrasonically dissolved in 0.20 mL of tetrahydrofuran to obtain a clear solution, i.e., solution 4a-1; 12.10 mg of 2-naphthalenesulfonic acid was dissolved in 0.65 mL of tetrahydrofuran to obtain solution 4b-1; at room temperature with stirring, solution 4b-1 was added dropwise to solution 4a-1 to obtain solution 4c-1; solution 4c-1 was continuously stirred for 2 h, and then a solid was precipitated to obtain a suspension; the suspension was stirred overnight and then centrifuged; and the resulting solid was dried under vacuum at room temperature to obtain the 2-naphthalenesulfonate of compound A. The compound was identified as a crystalline form by XRPD and named as crystal form II of 2-naphthalenesulfonate of compound A.

¹H NMR (400 MHz, DMSO-*d6*) δ 10.48 (s, 1H), 8.84 - 8.77 (m, 1H), 8.24 (d, 1H), 8.19 - 8.10 (m, 3H), 7.96 (t, 1H), 7.93 - 7.84 (m, 2H), 7.80 (d, 1H), 7.72 (dd, 1H), 7.66 - 7.49 (m, 5H), 7.47 - 7.28 (m, 3H), 5.93 (d, 1H), 5.05 - 4.85 (m, 1H), 4.50 (d, 1H), 3.72 (d, 1H), 3.08 (t, , 1H), 2.86 (d, 2H), 2.45 (d, 3H), 2.12 - 1.94 (m, 1H), 1.76 - 1.60 (m, 1H), 1.45 - 1.00 (m, 5H).

The crystal form I of 2-naphthalenesulfonate of compound **A** obtained in example 4 is a crystalline form as identified by XRPD. Table 5 shows the XRPD peak list; Figure 11 shows the XRPD pattern; and Figure 13 shows the TGA pattern exhibiting a weight loss of 12.17% below 150°C and exhibiting a decomposition temperature of 211.99°C. Figure 12 shows the DSC pattern, with a melting point of approximately 143.43°C.

The crystal form I of 2-naphthalenesulfonate of compound **A** obtained in example 4 is a crystalline form as identified by XRPD. Table 6 shows the XRPD peak list; and Figure 14 shows the XRPD pattern.

### Example 5: Preparation of benzenesulfonate of compound A

At room temperature, 90 mg of the amorphous compound A was dissolved in 7.50 mL of isopropanol to obtain solution 5a; 30.0 mg of benzenesulfonic acid was dissolved in 0.48 mL of isopropanol to obtain solution 5b; at room temperature with stirring, solution 5b was added dropwise to solution 5a to obtain solution 5c; solution 5c was continuously stirred for 1 h, and then a solid was precipitated to obtain a suspension; the suspension was stirred overnight and then centrifuged; and the resulting solid was dried under vacuum at room temperature to obtain the benzenesulfonate of compound A.

¹H NMR (500 MHz, DMSO-*d6*) δ 10.50 (s, 1H), 8.80 (t, 1H), 8.25 (d, 1H), 8.19 - 8.11 (m, 2H), 7.80 (d, 1H), 7.67 - 7.53 (m, 5H), 7.46 - 7.25 (m, 6H), 5.93 (dd, 1H), 5.02 - 4.74 (m, 1H), 4.49 (s, 1H), 3.71 (d, 1H), 3.07 (t, 1H), 2.84 (d, 2H), 2.46 (d, 3H), 2.09 - 1.98 (m, 1H), 1.76 - 1.61 (m, 1H), 1.45 - 1.11 (m, 5H).

### Example 6: Preparation of methanesulfonate of compound A

At room temperature, 90 mg of the amorphous compound A was dissolved in 0.60 mL of acetone to obtain solution 6a; 18.0 mg of methanesulfonic acid was dissolved in 1.80 mL of acetone to obtain solution 6b; at room temperature with stirring, solution 6b was added dropwise to solution 6a to obtain solution 6c; solution 6c was continuously stirred for a few minutes, and then a solid was precipitated to obtain a suspension; the suspension was stirred overnight and then centrifuged; and the resulting solid was dried under vacuum at room temperature to obtain the methanesulfonate of compound A.

¹H NMR (500 MHz, DMSO-*d6*) δ 10.50 (s, 1H), 8.80 (t, 1H), 8.25 (d, 1H), 8.18 - 8.11 (m, 2H), 7.80 (d, 1H), 7.66 - 7.53 (m, 3H), 7.46 - 7.29 (m, 3H), 5.93 (dd, 1H), 5.05 - 4.85 (m, 1H), 4.49 (s, 1H), 3.71 (d, 1H), 3.07 (t, 1H), 2.84 (d, 2H), 2.45 (d, 3H), 2.38 (s, 3H), 2.11 - 2.00 (m, 1H), 1.72 - 1.62 (m, 1H), 1.44 - 1.11 (m, 5H).

### Example 7: Preparation of p-toluenesulfonate of compound A

At room temperature, approximately 90 mg of the amorphous compound A was dissolved in 0.60 mL of acetone to obtain solution 7a; 29.58 mg of p-toluenesulfonic acid was dissolved in 2.04 mL of acetone to obtain solution 7b; at room temperature with stirring, solution 7b was added dropwise to solution 7a to obtain solution 7c; solution 7c was continuously stirred, and then a solid was precipitated to obtain a suspension; the suspension was stirred overnight and then centrifuged; and the resulting solid was dried under vacuum at room temperature to obtain the p-toluenesulfonate of compound A.

¹H NMR (500 MHz, DMSO-*d6*) δ 10.50 (s, 1H), 8.80 (t, 1H), 8.25 (d, 1H), 8.18 - 8.11 (m, 2H), 7.80 (d, 1H), 7.65 - 7.59 (m, 2H), 7.56 (d, 1H), 7.51 - 7.45 (m, 2H), 7.45 - 7.30 (m, 3H), 7.12 (d, 2H), 5.93 (dd, 1H), 5.02 - 4.73 (m, 1H), 4.49 (s, 1H), 3.71 (d, 1H), 3.07 (t, 1H), 2.84 (d, 2H), 2.46 (d, 3H), 2.29 (s, 3H), 2.08 - 2.00 (m, 1H), 1.72 - 1.62 (m, 1H), 1.46 - 1.07 (m, 5H).

### Example 8: Preparation of acetate, fumarate, malonate, succinate, benzoate, citrate, malate and L-tartrate of compound A

With reference to the methods in Examples 1-7, according to the feeding ratios in Table 7, the corresponding salts were prepared.

**Table 7 Salt formation from compound A with different acids**

| Acid | **Solvent for free compound A** | **Solvent for acid** | **Feeding molar ratio (base : acid)** | **Anti-solvent** |
|---|---|---|---|---|
| Acetic acid | Isopropanol | Isopropanol | 1 : 1.2 | - |
| | Methanol | Methanol | 1 : 1.2 | - |
| | - | Acetic acid : water = 1 : 1.2 | - | - |
| | - | Acetic acid : methyl tert-butyl ether = 1 : 2 | - | - |
| Fumaric acid | Isopropanol | Isopropanol | 1 : 1.2 | Isopropyl ether |
| | Acetone | Acetone, water | 1 : 1.2 | Isopropyl ether |
| | Tetrahydrofuran | Tetrahydrofuran, water | 1 : 1.2 | n-Heptane |
| | Ethanol | Ethanol | 1 : 1.2 | - |
| Malonic acid | Isopropanol | Isopropanol | 1 : 1.2 | - |
| | Acetone | Acetone | 1 : 1.2 | Isopropyl ether |
| | Tetrahydrofuran | Tetrahydrofuran | 1 : 1.2 | n-Heptane |
| | Ethanol | Ethanol | 1 : 1.2 | - |
| | Methanol | Methanol | 1 : 1.2 | - |
| | Acetone | Acetone | 1 : 1.2 | - |
| | Tetrahydrofuran | Tetrahydrofuran/w ater | 1 : 1.2 | n-Heptane |
| | Ethanol | Ethanol | 1 : 1.2 | - |
| | Acetone | Acetone | 1 : 1.2 | - |
| | Methanol | Methanol | 1 : 1.2 | - |
| Benzoic acid | Isopropanol | Isopropanol | 1 : 1.2 | Isopropyl ether |
| | Acetone | Acetone | 1 : 1.2 | Isopropyl ether |
| | Tetrahydrofuran | Tetrahydrofuran | 1 : 1.2 | n-Heptane |
| | Ethanol | Ethanol | 1 : 1.2 | - |
| | Acetone | Acetone | 1 : 1.2 | - |
| | Methanol | Methanol | 1 : 1.2 | - |
| | Acetone | Acetone | 1 : 1.2 | - |
| | Tetrahydrofuran | Tetrahydrofuran | 1 : 1.2 | n-Heptane |
| | Ethanol | Ethanol | 1 : 1.2 | - |
| | Tetrahydrofuran | Tetrahydrofuran | 1 : 1.2 | n-Heptane |
| | Ethanol | Ethanol | 1 : 1.2 | - |
| L-tartaric acid | Isopropanol | Isopropanol | 1 : 1.2 | Isopropyl ether |
| | Acetone | Acetone | 1 : 1.2 | Isopropyl ether |
| | Tetrahydrofuran | Tetrahydrofuran | 1 : 1.2 | n-Heptane |

Test results: no salt form or stable salt form was prepared.

### 1. Characterization of salt form of compound A

The properties of various salts of compound A are shown in Table 8.

**Table 8 Properties of various salts of compound A**

| **Name** | **Characterization of crystal form** | **Crystallinity** | **Category** | **Molar ratio for salt formation (base: acid)** | **Melting point (°C)** | **Crystal form at high humidity** | **PLM (hot-stage polarized light microscopy)** |
|---|---|---|---|---|---|---|---|
| Free base | Amorphous | Low | - | - | - | - | Tabular particles |
| Hemi-1,5-naphthalene disulfonate | Crystal form 1 | High | Anhydrid e | 1 : 0.5 | 189 | Crystal form unchanged | Agglomerated particles |
| Hydrochloride | Crystal form 1 | High | Solvate | 1 : 1 | 119 | Crystal form unchanged | Agglomerated particles |
| Hydrobromide | Crystal form 1 | High | Solvate | 1 : 1 | 180 | - | Agglomerated particles |
| | Crystal form 2 | High | - | - | - | - | - |
| 2-naphthalenesulfo nate | Crystal form 1 | High | Anhydrid e | 1 : 1 | 143 | - | Agglomerated particles |
| | Crystal form 2 | High | - | - | - | - | - |
| Benzenesulfonate | Benzenesulfonate 1: | Relatively low | - | 1 : 1 | - | - | Tabular particles |
| p-toluenesulfonate | - | Relatively low | - | 1 : 1 | - | - | Blocky particles |
| Methanesulfonate | - | Low | - | 1 : 1 | - | - | Blocky particles |

| | | | | | | | |
|---|---|---|---|---|---|---|---|
| Notes: crystallinity is determined by the height of the strongest peak in XRPD; and melting points are determined by DSC and expressed as onset values. | | | | | | | |

### 2. Stability study:

Samples: crystal form I of hemi-1,5-naphthalene disulfonate of compound **A** in Example 1, crystal form I of hydrochloride of compound **A** in Example 2, crystal form I of hydrobromide of compound **A** in Example 3, and crystal form I of 2-naphthalenesulfonate of compound **A** in Example 4.

Experiments: the crystal form I of hemi-1,5-naphthalene disulfonate of compound **A** in Example 1, crystal form I of hydrochloride of compound **A** in Example 2, crystal form I of hydrobromide of compound **A** in Example 3, and crystal form I of 2-naphthalenesulfonate of compound **A** in Example 4 were respectively placed at an accelerated condition (open, 40°C, 75% RH (relative humidity)), a light condition (open, 25°C, total illuminance not less than 1.2 × 106 Lux.hr, near-ultraviolet energy not less than 200 w.hr/m2), a high humidity condition (open, 60°C, drying oven) and a long-term condition (open, 25°C-60% RH) for stability experiments. On day 0, day 5, day 10 and day 15, the samples were taken and detected for purity by HPLC (expressed as a percentage). The experimental results are shown in Table 9.

Conditions for detecting purity by HPLC: chromatographic column: ChromCore 120 C18 5 µm (4.6 mm* 100); column temperature: 35°C; detection wavelength: 220 nm; mobile phase: 10 mmol/L ammonium formate aqueous solution: acetonitrile = 55 : 45 (v/v); flow rate: 1.0 mL/min.

**Table 9 HPLC results of experiments for solid state stability study**

| **Sample/influencing factor** | **Initial value** | **Long-term condition** | | **High temperature condition** | | **Accelerated condition** | | | **Light condition** | |
|---|---|---|---|---|---|---|---|---|---|---|
| Time | Day 0 | Day 5 | Day 10 | Day 5 | Day 10 | Day 5 | Day 10 | Day 15 | Day 5 | Day 10 |
| Crystal form I of hemi-1,5-naphthalene disulfonate of compound **A** in Example 1 | 98.80 | 98.50 | 98.47 | 98.52 | 98.28 | - | - | - | 98.90 | 98.62 |
| Crystal form I of hydrochloride of compound **A** in Example 2 | 98.95 | 98.42 | 98.12 | 98.57 | 98.53 | - | - | - | 99.00 | 98.91 |
| Crystal form I of hydrobromide of compound **A** in Example 3 | 99.35 | 99.32 | 99.25 | 99.27 | 99.29 | - | - | - | 99.10 | 98.83 |
| Crystal form I of 2-naphthalenesulfonat e of compound **A** in Example 4 | 99.46 | 99.41 | 99.39 | 99.36 | 99.38 | 98.94 | 98.43 | 97.82 | 99.45 | 99.37 |

Conclusion: after placed at a high temperature condition, a long-term condition or a light condition for 10 days, the compounds of Examples 1-4 have basically no change in purity and little change in single impurity content, indicating good stability; in addition, a stability test at an accelerated condition was performed on the crystal form I of 2-naphthalenesulfonate of compound A in Example 4, which showed good stability.

### 3. Pharmacokinetics of example compounds

### 3.1. Pharmacokinetic test in rats

Test objective: by giving test compounds to SD rats via single-dose intravenous and intragastric administration and measuring the concentrations of the test compounds in plasma of rats, the pharmacokinetic characteristics and bioavailability of the test compounds in rats were evaluated.

Test compound: compound A, crystal form I of hemi-1,5-naphthalene disulfonate of compound A in Example 1 and crystal form I of 2-naphthalenesulfonate of compound A in Example 4.

Test animal: male SD rats, about 220 g, 6-8 weeks old, 6 rats/compound, purchased from Chengdu Ddossy Experimental Animals Co., Ltd.

Test method: on the day of the test, 6 SD rats were randomly grouped according to their body weight; the animals were fasted with water available for 12 to 14 h one day before the administration, and were fed 4 h after the administration; and the administration was performed according to Table 10.

**Table 10 Administration information**

| Group | Number | Administration information | | | | | | |
|---|---|---|---|---|---|---|---|---|
| | Male | Test compound | Administratio n dosage* (mg/kg) | Administration concentration (mg/mL) | Administration volume (mL/kg) | Collected sample | Mode of administration | Vehicle |
| G1 | 3 | Compound A or a salt thereof | 2 | 0.4 | 5 | Plasma | Intravenously | 5% DMA+95 %(20% SBE-β-CD) |
| G2 | 3 | Compound A or a salt thereof | 10 | 1 | 10 | Plasma | Intragastrically | 0.5%MC |

| | | | | | | | | |
|---|---|---|---|---|---|---|---|---|
| *Dosage is calculated on the basis of free base. | | | | | | | | |

### Biological sample collection

Before and after the administration, 0.1 mL of blood samples were drawn from the orbits of the animals under isoflurane anaesthesia, and placed in an EDTAK2 centrifuge tube. Centrifugation was performed at 5000 rpm at 4°C for 10 min, and plasma was collected.

Time points for sample collection in G1 and G2 groups comprise 0, 5 min, 15 min, 30 min, 1 h, 2h, 4h, 6 h, 8 h and 24 h.

Before analysis and detection, all samples were stored at -80°C.

### Pretreatment of samples

30 µL of each of plasma samples, standard curve samples and quality control samples was taken, and 200 µL of acetonitrile solution containing an internal standard was added. The resulting mixture was homogeneously mixed by vortex and centrifuged at 4°C at 12000 rpm for 10 min. 170 µL of the supernatant was taken and placed to a 96-well plate, and LC-MS/MS analysis was performed, wherein the sample size was 0.2 µL.

The main pharmacokinetic parameters were analysed by a non-compartmental model using WinNonlin 8.0 software. The test results were as shown in Table 11.

**Table 11 Pharmacokinetic parameters in rats**

| Test compound | Mode of administration | CO or Cmax (ng/ml) | AUC (h^{∗}ng/ml) | T_{1/2} (h) | Tmax (h) | F% |
|---|---|---|---|---|---|---|
| Compound A | IV 2 mg/kg | 1460 ± 134 | 2006 ± 396 | 0.897 ± 0.013 | - | - |
| | PO 10 mg/kg | 715 ± 127 | 2614 ± 620 | 1.04 ± 0.099 | 1.67 ± 0.58 | 26.1 ± 6.2 |
| Crystal form I of hemi-1,5-naphthalene disulfonate of compound A in Example 1 | IV 2 mg/kg | 1729 ± 215 | 2157 ± 509 | 1.18 ± 0.36 | - | - |
| | PO 10 mg/kg | 1393 ± 497 | 4521 ± 1682 | 1.38 ± 0.079 | 1.00 ± 0.0 | 41.9 ± 16 |
| Crystal form I of 2-naphthalenesulfon ate of compound A in Example 4 | IV 2 mg/kg | 1408 ± 83 | 1909 ± 846 | 1.05 ± 0.42 | - | - |
| | PO 10 mg/kg | 1190 ± 430 | 4209 ± 2090 | 1.42 ± 0.30 | 1.33 ± 0.58 | 44.1 ± 22 |

Conclusion: the salt forms of the compounds of the present application, such as crystal form I of hemi-1,5-naphthalene disulfonate of compound A in Example 1 and crystal form I of 2-naphthalenesulfonate of compound A in Example 4, have good pharmacokinetics in rats and significantly improved bioavailability compared with compound A in a free state.

### 3.2. Pharmacokinetic test in ferrets

Test objective: by giving test compounds to ferrets via single-dose intragastric administration, collecting plasma at different time points and measuring the concentrations of the test compounds in plasma of ferrets, the absorption of the test compounds in ferrets was evaluated in this test.

Test compound: crystal form I of hemi-1,5-naphthalene disulfonate of compound A in Example 1.

Test animal: 6 healthy adult male ferrets, about 800-1500 g, 6-10 months old, purchased from Wuxi Sangosho Biotechnology Co., Ltd.

Test method: the male ferrets selected for the test were fasted with water available for 12-14 h one day before the administration, and were fed 4 h after the administration; and the administration was performed according to Table 12.

**Table 12 Administration information**

| Group | Number | Administration information | | | | | | |
|---|---|---|---|---|---|---|---|---|
| | M | Test compound | Administration dosage (mg/kg) | Administration concentration (mg/mL) | Administration volume (mL/kg) | Collected sample | Mode of administration | Vehicle |
| G1 | 3 | Crystal form I of hemi-1,5-naphthalene disulfonate of compound **A** in Example 1 | 15 | 1.5 | 10 | Plasma | Intragastrically | 20% SBE-β-CD |
| G2 | 3 | Crystal form I of hemi-1,5-naphthalene disulfonate of compound **A** in Example 1 | 15 | 1.5 | 10 | Plasma | Intragastrically | 5% DMSO+ 5% Solutol+ 90% (0.5% MC) |

| | | | | | | | | |
|---|---|---|---|---|---|---|---|---|
| *Administration dosage is calculated on the basis of free base. | | | | | | | | |

Time points for blood collection: before the administration and 0.0833 h, 0.25 h, 0.5 h, 1 h, 2 h, 4 h, 6 h, 8 h and 24 h after the administration (10 time points in total), venous blood samples were collected into centrifuge tubes and centrifuged within 30 minutes, and the centrifuged plasma samples were stored in a refrigerator at -80°C for PK analysis.

The test results were as shown in Table 13.

**Table 13 Pharmacokinetic parameters in ferrets**

| Example no. | Mode of administration | CO or Cmax (ng/ml) | AUC (h*ng/ml) | T_{1/2} (h) | Tmax (h) | Vehicle |
|---|---|---|---|---|---|---|
| Crystal form I of hemi-1,5-naphthalene disulfonate of compound A in Example 1 | PO 15 mg/kg | 314 ± 32 | 393 ± 76 | 1.13 ± 0.43 | 0.583 ± 0.38 | 20% SBE-β-CD |
| Crystal form I of hemi-1,5-naphthalene disulfonate of compound A in Example 1 | PO 15 mg/kg | 942 | 1354 | 0.705 | 0.750 | 5% DMSO+ 5% Solutol+ 90% (0.5% MC) |

Conclusion: the salt forms of the compounds of the present application, such as crystal form I of hemi-1,5-naphthalene disulfonate of compound A in Example 1, have good pharmacokinetics in ferrets.

## Claims

1. A salt of compound A or a hydrate or solvate of a salt thereof, 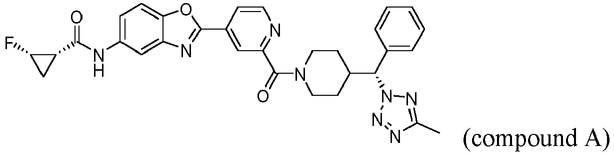 wherein the salt is hydrochloride, hydrobromide, 2-naphthalenesulfonate, benzenesulfonate, methanesulfonate, p-toluenesulfonate, hemi-1,5-naphthalene disulfonate, succinate, citrate or malate.

2. The salt or the hydrate or solvate of the salt thereof according to claim 1, wherein the salt is hydrochloride, hydrobromide, 2-naphthalenesulfonate or hemi-1,5-naphthalene disulfonate, preferably the salt thereof exists in the form of a crystal.

3. The salt or the hydrate or solvate of the salt thereof according to claim 2, wherein the salt is hemi-1,5-naphthalene disulfonate of compound A, which is in the form of a crystal and has an X-ray powder diffraction pattern comprising characteristic diffraction peaks at 5.3° ± 0.2°, 13.4° ± 0.2°, 17.4° ± 0.2°, 18.5° ± 0.2°, 20.4° ± 0.2° and 23.6° ± 0.2° 2θ, as determined by using Cu-Kα radiation.

4. The salt or the hydrate or solvate of the salt thereof according to claim 3, having an X-ray powder diffraction pattern further comprising characteristic diffraction peaks at 7.0° ± 0.2°, 9.8° ± 0.2°, 10.6° ± 0.2°, 12.7° ± 0.2°, 14.8° ± 0.2°, 22.2° ± 0.2° and 23.1° ± 0.2° 2θ.

5. The salt or the hydrate or solvate of the salt thereof according to claim 4, having an X-ray powder diffraction pattern further comprising characteristic diffraction peaks at 14.1° ± 0.2°, 16.0° ± 0.2° and 21.5° ± 0.2° 2θ.

6. The salt or the hydrate or solvate of the salt thereof according to claim 5, having an X-ray powder diffraction pattern substantially as shown in Figure 1.

7. The salt or the hydrate or solvate of the salt thereof according to claim 2, wherein the salt is hydrochloride of compound A, which is in the form of a crystal and has an X-ray powder diffraction pattern comprising characteristic diffraction peaks at 5.9° ± 0.2°, 11.2° ± 0.2°, 11.7° ± 0.2°, 17.6° ± 0.2°, 18.2° ± 0.2°, 21.9° ± 0.2° and 26.8° ± 0.2° 2θ, as determined by using Cu-Kα radiation.

8. The salt or the hydrate or solvate of the salt thereof according to claim 7, having an X-ray powder diffraction pattern further comprising characteristic diffraction peaks at 7.1° ± 0.2°, 16.3° ± 0.2°, 18.6° ± 0.2°, 22.3° ± 0.2° and 23.8° ± 0.2° 2θ.

9. The salt or the hydrate or solvate of the salt thereof according to claim 8, having an X-ray powder diffraction pattern further comprising characteristic diffraction peaks at 13.3° ± 0.2°, 14.2° ± 0.2°, 15.7° ± 0.2°, 20.3° ± 0.2°, 21.3° ± 0.2°, 24.8° ± 0.2°, 25.4° ± 0.2°, 27.2° ± 0.2° and 27.7° ± 0.2° 2θ.

10. The salt or the hydrate or solvate of the salt thereof according to claim 9, having an X-ray powder diffraction pattern substantially as shown in Figure 4.

11. The salt or the hydrate or solvate of the salt thereof according to claim 2, wherein the salt is hydrobromide of compound A, which is in the form of a crystal and has an X-ray powder diffraction pattern comprising characteristic diffraction peaks at 6.0° ± 0.2°, 7.2° ± 0.2°, 9.0° ± 0.2°, 12.0° ± 0.2°, 14.8° ± 0.2° and 17.6° ± 0.2° 2θ, as determined by using Cu-Kα radiation.

12. The salt or the hydrate or solvate of the salt thereof according to claim 11, having an X-ray powder diffraction pattern further comprising characteristic diffraction peaks at 17.3° ± 0.2°, 18.0° ± 0.2°, 21.2° ± 0.2°, 21.5° ± 0.2°, 24.2° ± 0.2° and 26.5° ± 0.2° 2θ.

13. The salt or the hydrate or solvate of the salt thereof according to claim 12, having an X-ray powder diffraction pattern further comprising characteristic diffraction peaks at 16.9° ± 0.2°, 18.6° ± 0.2°, 19.0° ± 0.2°, 20.2° ± 0.2° and 28.0° ± 0.2° 2θ.

14. The salt or the hydrate or solvate of the salt thereof according to claim 13, having an X-ray powder diffraction pattern substantially as shown in Figure 7.

15. The salt or the hydrate or solvate of the salt thereof according to claim 2, wherein the salt is hydrobromide of compound A, which is in the form of a crystal and has an X-ray powder diffraction pattern comprising characteristic diffraction peaks at 6.5° ± 0.2°, 7.3° ± 0.2°, 12.2° ± 0.2°, 12.9° ± 0.2° and 16.0° ± 0.2° 2θ, as determined by using Cu-Kα radiation.

16. The salt or the hydrate or solvate of the salt thereof according to claim 15, having an X-ray powder diffraction pattern further comprising characteristic diffraction peaks at 6.1° ± 0.2°, 17.4° ± 0.2°, 18.3° ± 0.2°, 20.4° ± 0.2°, 22.4° ± 0.2°, 24.8° ± 0.2° and 28.2° ± 0.2° 2θ.

17. The salt or the hydrate or solvate of the salt thereof according to claim 16, having an X-ray powder diffraction pattern further comprising characteristic diffraction peaks at 13.8° ± 0.2°, 14.5° ± 0.2°, 15.2° ± 0.2°, 19.1° ± 0.2°, 19.9° ± 0.2°, 21.4° ± 0.2°, 21.8° ± 0.2°, 23.1° ± 0.2°, 23.6° ± 0.2°, 25.5° ± 0.2°, 26.0° ± 0.2° and 26.5° ± 0.2° 2θ.

18. The salt or the hydrate or solvate of the salt thereof according to claim 17, having an X-ray powder diffraction pattern substantially as shown in Figure 10.

19. The salt or the hydrate or solvate of the salt thereof according to claim 2, wherein the salt is 2-naphthalenesulfonate of compound A, which is in the form of a crystal and has an X-ray powder diffraction pattern comprising characteristic diffraction peaks at 4.7° ± 0.2°, 9.4° ± 0.2°, 17.2° ± 0.2°, 21.2° ± 0.2° and 23.4° ± 0.2° 2θ, as determined by using Cu-Kα radiation.

20. The salt or the hydrate or solvate of the salt thereof according to claim 19, having an X-ray powder diffraction pattern further comprising characteristic diffraction peaks at 6.3° ± 0.2°, 6.8° ± 0.2°, 7.8° ± 0.2°, 13.4° ± 0.2°, 16.5° ± 0.2°, 19.2° ± 0.2° and 20.1° ± 0.2° 2θ.

21. The salt or the hydrate or solvate of the salt thereof according to claim 20, having an X-ray powder diffraction pattern further comprising characteristic diffraction peaks at 14.9° ± 0.2°, 15.3° ± 0.2°, 15.7° ± 0.2°, 24.3° ± 0.2°, 25.1° ± 0.2° and 26.1° ± 0.2° 2θ.

22. The salt or the hydrate or solvate of the salt thereof according to claim 21, having an X-ray powder diffraction pattern substantially as shown in Figure 11.

23. The salt or the hydrate or solvate of the salt thereof according to claim 2, wherein the salt is 2-naphthalenesulfonate of compound A, which is in the form of a crystal and has an X-ray powder diffraction pattern comprising characteristic diffraction peaks at 5.6° ± 0.2°, 11.2° ± 0.2°, 14.1° ± 0.2°, 16.0° ± 0.2°, 22.8° ±0.2° and 26.8° ± 0.2° 2θ, as determined by using Cu-Kα radiation.

24. The salt or the hydrate or solvate of the salt thereof according to claim 23, having an X-ray powder diffraction pattern further comprising characteristic diffraction peaks at 4.5° ± 0.2°, 6.2° ± 0.2°, 6.8° ± 0.2°, 8.4° ± 0.2°, 10.4° ± 0.2°, 15.3° ± 0.2°, 15.6° ± 0.2°, 19.0° ± 0.2°, 19.6° ± 0.2° and 25.5 ± 0.2° 2θ.

25. The salt or the hydrate or solvate of the salt thereof according to claim 24, having an X-ray powder diffraction pattern further comprising characteristic diffraction peaks at 12.4° ± 0.2°, 12.7° ± 0.2°, 16.7° ± 0.2°, 17.2° ± 0.2°, 17.5° ± 0.2°, 18.0° ± 0.2°, 20.8° ± 0.2°, 21.8° ± 0.2°, 23.5° ± 0.2° and 24.3° ± 0.2° 2θ.

26. The salt or the hydrate or solvate of the salt thereof according to claim 25, having an X-ray powder diffraction pattern substantially as shown in Figure 14.

27. A method for preparing the hemi-1,5-naphthalene disulfonate of compound A, comprising the steps of
(1) dissolving amorphous compound A in solvent 1;
(2) dissolving 1,5-naphthalene disulfonic acid in solvent 2;
(3) adding dropwise a solution obtained in step (2) to a solution obtained in step (1) at room temperature with stirring; and stirring a reaction;
wherein the solvent 1 is selected from one of isopropanol, acetone or tetrahydrofuran, and the solvent 2 is a single-solvent system or a double-solvent mixed system, wherein the single-solvent system is selected from one of isopropanol, acetone or tetrahydrofuran, and the double-solvent mixed system is a tetrahydrofuran-water mixed liquid.

28. A method for preparing the 2-naphthalenesulfonate of compound A, comprising the steps of
(1) dissolving amorphous compound A in solvent 3;
(2) dissolving 2-naphthalenesulfonic acid in solvent 4;
(3) adding dropwise a solution obtained in step (2) to a solution obtained in step (1) at room temperature with stirring; and carrying out stirring, crystallization, centrifugation and drying;
wherein the solvent 3 is selected from one of isopropanol, acetone, tetrahydrofuran, isopropyl acetate, 1,4-dioxane, toluene or dimethyl sulfoxide, and the solvent 4 is a single-solvent system or a double-solvent mixed system, wherein the single-solvent system is selected from one of isopropanol, acetone, tetrahydrofuran, isopropyl acetate, 1,4-dioxane, toluene or dimethyl sulfoxide, and the double-solvent mixed system is a toluene-methanol mixed liquid.

29. A method for preparing the hydrochloride of compound A, comprising the steps of
(1) dissolving amorphous compound A in solvent 5;
(2) dissolving hydrochloric acid in solvent 6;
(3) adding dropwise a solution obtained in step (2) to a solution obtained in step (1) at room temperature with stirring; and stirring a reaction;
wherein the solvent 5 and solvent 6 are each independently selected from one of isopropanol, acetone and tetrahydrofuran.

30. A method for preparing the hydrobromide of compound A, comprising the steps of
(1) dissolving amorphous compound A in solvent 7;
(2) dissolving hydrobromic acid in solvent 8;
(3) adding dropwise a solution obtained in step (2) to a solution obtained in step (1) at room temperature with stirring; and stirring a reaction;
wherein the solvent 7 and solvent 8 are each independently selected from one of isopropanol, acetone and tetrahydrofuran.

31. A pharmaceutical composition comprising a therapeutically effective amount of the salt or the hydrate or solvate of the salt thereof according to any one of claims 1 to 26, and a pharmaceutically acceptable carrier or excipient.

32. The salt or the hydrate or solvate of the salt thereof according to any one of claims 1 to 26 or the pharmaceutical composition according to claim 31 in the preparation of a medicament for preventing and/or treating influenza.

33. A method for preventing and/or treating influenza, comprising administering to a subject in need thereof a therapeutically effective amount of the salt or the hydrate or solvate of the salt thereof according to any one of claims 1 to 26 or the pharmaceutical composition according to claim 31.

34. The salt or the hydrate or solvate of the salt thereof according to any one of claims 1 to 26 or the pharmaceutical composition according to claim 31, for use in the prevention and/or treatment of influenza.

35. A composition for preventing and/or treating influenza, comprising the salt or the hydrate or solvate of the salt thereof according to any one of claims 1 to 26.
